# EUROPEAN PATENT SPECIFICATION

(11) **EP 2 095 826 B1**
(45) Date of publication and mention of the grant of the patent: **26.12.2012**
(21) Application number: 07832014.0
(22) Date of filing: 16.11.2007
(51) Int. Cl.: A61K 39/395, A61P 21/04, A61P 25/00, A61P 25/04, A61P 25/16, A61P 25/28, A61P 25/30, A61P 35/00, C07K 16/08

(54) **NERVE ELONGATION PROMOTER AND ELONGATION INHIBITOR**
NERVENELONGATIONSPROMOTER UND ELONGATIONSHEMMER
AGENT PROMOTEUR DE L'ETIREMENT DES NERFS ET AGENT INHIBITEUR DE L'ETIREMENT DES NERFS

(30) Priority: 17.11.2006 JP 2006312236
(43) Date of publication of application: 02.09.2009
(73) Proprietor: The Research Foundation for Microbial Diseases of Osaka University, Suita-shi, Osaka 565-0871 (JP)
(72) Inventor: SHIRAKI, Kimiyasu, Toyama-shi Toyama 930-0194 (JP); HAMA, Yuka, Toyama-shi Toyama 930-0194 (JP); YOSHIDA, Yoshihiro, Toyama-shi Toyama 930-0194 (JP); TSUDA, Masaaki, Toyama-shi Toyama 930-0194 (JP); TSUMOTO, Tadaharu, Wako-shi Saitama 351-0198 (JP); ENDO, Shunro, Toyama-shi Toyama 930-0194 (JP); TAKAHASHI, Michiaki, Suita-shi Osaka 565-0871 (JP)
(74) Representative: Duckworth, Timothy John
(86) International application number: PCT/JP2007/072284
(87) International publication number: WO 2008/059959

(56) References cited:
- WO-A1-95/04080
- JP-A- 2002 512 627
- KINCHINGTON P R ET AL: "Virion association of IE62, the varicella-zoster virus (VZV) major transcriptional regulatory protein, requires expression of the VZV open reading frame 66 protein kinase." JOURNAL OF VIROLOGY OCT 2001 LNKD- PUBMED:11533174, vol. 75, no. 19, October 2001 (2001-10), pages 9106-9113, XP002606402 ISSN: 0022-538X
- GARRY EMER M ET AL: "Varicella zoster virus induces neuropathic changes in rat dorsal root ganglia and behavioral reflex sensitisation that is attenuated by gabapentin or sodium channel blocking drugs." PAIN NOV 2005 LNKD- PUBMED:16213091, vol. 118, no. 1-2, November 2005 (2005-11) , pages 97-111, XP002606403 ISSN: 0304-3959
- GILDEN DONALD H ET AL: "VZV vasculopathy and postherpetic neuralgia: progress and perspective on antiviral therapy." NEUROLOGY 11 JAN 2005 LNKD- PUBMED:15642898, vol. 64, no. 1, 11 January 2005 (2005-01-11), pages 21-25, XP002606404 ISSN: 1526-632X
- HAMA YUKA ET AL: "Antibody to varicella-zoster virus immediate-early protein 62 augments allodynia in zoster via brain-derived neurotrophic factor." JOURNAL OF VIROLOGY FEB 2010 LNKD- PUBMED:19923188, vol. 84, no. 3, February 2010 (2010-02), pages 1616-1624, XP002606409 ISSN: 1098-5514
- HAMA Y.: 'Suiho Obi-jo Hosshin Virus (VZV) no Zen Shoki Tanpaku IE62 to Shinkei Inshi tono Kanren ni tsuite' CLINICAL VIROLOGY vol. 35, no. 2, 21 May 2007, page S94, XP003025828
- LOWRY P.W. ET AL.: 'Immunity in strain 2 guinea-pigs inoculated with vaccinia virus recombinants expressing varicella-zoster virus glycoproteins I, IV, V or the protein product of the immediate early gene 62' J. GENE VIROL. vol. 73, no. PART 4, 1992, pages 811 - 819, XP001056690
- HUANG L. ET AL.: 'Glial cell line-derived neurotrophic factor (GDNF) is required for differentiation of pontine noradrenergic neurons and patterning of central respiratory output' NEUROSCIENCE vol. 130, no. 1, 2005, pages 95 - 105, XP025366611
- KIMPINSKI K. ET AL.: 'The anti-p75 antibody, MC192, and brain-derived neurotrophic factor inhibit nerve growth factor-dependent neurite growth from adult sensory neurons' NEUROSCIENCE vol. 93, no. 1, 1999, pages 253 - 263, XP008109933

## Description

### Technical Field

The present invention relates to a nerve elongation promoter and elongation inhibitor. Specifically, the present invention relates to a use of an antibody that recognizes a varicella-zoster virus immediate-early antigen for neurite elongation promotion, nerve regeneration or neurite elongation inhibition.

### Background Art

Brain-derived neurotrophic factor (BDNF) is a member of the neutrotrophin family that is recognized as playing an important role in the survival, differentiation, synaptic plasticity, and outgrowth of select peripheral and central neurons during development and in adulthood (patent document 1, non-patent documents 1 and 2). BDNF cDNA encodes a precursor protein consisting of 247 amino acid residues with a signal peptide and a proprotein. BDNF is cleaved to yield the 119 amino acid residue mature BDNF. Bioactive BDNF (27 kDa) is a dimer formed by two identical subunits held together by strong hydrophobic interactions. It is well known that BDNF participates in use-dependent plasticity mechanisms such as long-term potentiation of synapse, learning, and memory. BDNF is involved in the plasticity responsible for clinical pain hypersensitivity (non-patent document 3).

Varicella-zoster virus (VZV) infection causes neurological complications such as acute cerebellar ataxia (ACA) and postherpetic neuralgia (PHN). ACA is the most common neurological complication associated with varicella infection. It is estimated to occur in 1 in 4,000 cases of children below the age of 15. ACA appears from some days before to 3 weeks after onset of the rash and continues 2-4 weeks with complete recovery. Herpes zoster presents a vesicular rash in a dermatomal distribution with sensory abnormalities and its annual incidence in the general population was found to be 3.4/1,000 persons in the United Kingdom (non-patent document 4). PHN is the most frequent complication of Herpes zoster, occurring in 7-35% of patients (more than 50% older than 60). PHN differs from the pain associated with acute neuritis and combines constant pain, lancinating pain, and allodynia (non-patent document 5). Both neurological complications appear in or later than the convalescent phase, suggesting the immune response to VZV might participate in their pathogenic process. patent document 1: JP-A-5-328974
non-patent document 1: Nagappan G. Lu B., Trends in Neurosciences, 28: 464-471, 2005
non-patent document 2: Bramham CR et al., Progress in Neurology, 76: 99-125, 2005
non-patent document 3: Coull, J. A. M. et al., Nature 438: 1017-1021, 2005
non-patent document 4: Johnson, R. W. et al., BMJ 326: 748-750, 2003
non-patent document 5: Donald H. Gilden et al., Neurology 64: 21-25, 2005

Kinchington et al, J. Virol. 75(19), 9106-9113, Oct 2001, reports that IE62, the major transcriptional regulatory protein encoced by VZV, is associated with the tegument of gradient-purified virions. The association of IE62 with wild-type VZV virions was confirmed using immunoelectron microscopy with IE62-specific antibodies.

Emer et al, Pain 118,97-111, 2005, reports that VZV induces neuropathic changes in rat dorsal root ganglia and behavioural reflex sensitization that is attenuated by gabapentin or sodium channel blocking drugs.

Huang et al, Neuroscience 130(1), 95-105, 2005, shows that blocking antibodies against BDNF inhibit glial cell line-derived neurotrophic factor (GDNF) where GDNF is required for development of neurons *in vivo.*

Kimpinski *et al* reports that the anti-p75 antibody MC192 and BDNF inhibit nerve growth factor-dependent neurite growth from adult sensory neurons.

### Disclosure of the Invention

### Problems to Be Solved by the Invention

It is an object of the present invention to provide a means of contributing to the elucidation of the mechanism for post-zoster neuralgia, and promoting the regeneration of a damaged nerve. It is another object of the present invention to provide a means that is effective, with decreased adverse reactions, in preventing or treating disorders due to nerve cell death that accompanies post-zoster neuralgia, chronic pains, postherpetic neuralgia, cerebral stroke, degenerative diseases and the like, and the aforementioned diseases.

### Means of Solving the Problems

The present inventors hypothesized BDNF may participate in the pathogenesis of PHN and examined immunological relatedness of varicella-zoster virus with BDNF. VZV expresses genes 4, 21, 29, 62, 63 in latently infected ganglia and immediate early (IE) 62 protein plays a pivotal role in the transactivating viral gene during lytic infection. Zoster convalescent serum including PHN patients recognized IE62 and BDNF in Western blot analysis. The present inventors further examined the immunological relationship between IE62 and BDNF and found the immunological cross-reactivity between them. The significance of this immunological crossreaction has been elucidated by their respective monoclonal antibodies. The present inventors found that, among anti-IE62 antibodies cross-reacting to BDNF, some antibodies inhibited biological activities of BDNF, and others significantly augmented the signal transduction mediated by trkB mediated by BDNF and development of dendrites. As a result of the above, they have completed the present invention. Accordingly, the present invention provides the following.

[1] An agent for use in a method of promoting neurite elongation, comprising, as an active ingredient, an antibody that:
   - recognizes varicella-zoster virus immediate-early 62 protein,
   - recognizes a peptide having the amino acid sequence at the 414-429 positions of SEQ ID NO:2; and
   - cross-reacts with a brain-derived neurotrophic factor.
[2] The agent of [1] above, wherein the brain-derived neurotrophic factor with which the antibody cross-reacts is a dimer.
[3] An agent for use in a method of preventing or treating a nervous disease, comprising, as an active ingredient, an antibody as defined in [1] or [2] above.
[4] An antibody as defined in [1] or [2] above for use in a method of preventing or treating a nervous disease.
[5] The agent of [3] above or the antibody of [4] above, wherein the nervous disease is a disease with nerve cells damaged by a condition selected from cerebral stroke, anoxia, asphyxia, physical damage, exposure to toxins, malignant neoplasms, dementia, Alzheimer's disease, Parkinson's disease and amyotrophic lateral sclerosis.
[6] A commercial package comprising the agent of [1] or [2] above, and a written matter associated therewith, which states that the agent can or should be used for neurite elongation.
[7] A commercial package comprising the agent of [3] or [5] above, and a written matter associated therewith, which states that the agent can or should be used for a nervous disease.
[8] An agent for use in a method of inhibiting neurite elongation, comprising, as an active ingredient, an antibody that:
   - recognizes varicella-zoster virus immediate-early 62 protein,
   - recognizes a peptide having at least seven continuous amino acids selected from the amino acid sequence at the 268-341 positions of SEQ ID NO:2, and
   - cross-reacts with and inhibits a brain-derived neurotrophic factor.
[9] An agent for use in a method of preventing or treating a disease with nerve hypersensitivity caused by a condition selected from post-zoster neuralgia, chronic pains, experience-dependent social aversion and stress, which agent comprises, as an active ingredient, an antibody as defined in [8] above.
[10] An antibody as defined in [8] above for use in a method of preventing or treating a disease with nerve hypersensitivity caused by a condition selected from post-zoster neuralgia, chronic pains, experience-dependent social aversion and stress.
[11] A commercial package comprising the agent of [8] above, and a written matter associated therewith, which states that the agent can or should be used for inhibiting neurite elongation.
[12] A commercial package comprising the agent of [9] above, and a written matter associated therewith, which states that the agent can or should be used for a disease with nerve hypersensitivity caused by a condition selected from post-zoster neuralgia, chronic pains, experience-dependent social aversion and stress.
[13] A method of producing an antibody as defined in [1] or [2] above, comprising the steps of:
   - screening an antibody library using, as an antigen, a peptide having at least seven continuous amino acids selected from the amino acid sequence at the 414-429 positions of SEQ ID NO:2, and
   - screening using a brain-derived neurotrophic factor as a second antigen.
[14] A method of producing an antibody as defined in [8] above, comprising the steps of:
   - screening an antibody library using, as an antigen, a peptide having at least seven continuous amino acids selected from the amino acid sequence at the 268-341 positions of SEQ ID NO:2, and
   - screening using a brain-derived neurotrophic factor as a second antigen.
[15] The method of [13] or [14] above, wherein the antibody library is a human antibody library.

### Effect of the Invention

The agent for promoting neurite elongation of the present invention is expected to be efficiently effective in the presence of even a small amount of BDNF because of the ability thereof to promote neurite elongation by enhancing the action of BDNF. The antibody contained in the aforementioned promoter, compared with BDNF itself, has a longer half-life in a living organism, the BDNF-enhancing action persists, the autocrine system of BDNF is activated, the production and secretion of BDNF themselves are promoted, and the BDNF activity acting persistently on nerve cells rises. With further elapse of time, the activity of BDNF itself decreases; however, thanks to the persistent effect produced by the antibody with the longer half-life, it is possible to continue to increase the action of BDNF. According to the prophylactic or therapeutic agent for a nervous disease of the present invention, it is possible to regenerate nerves after nerve damage, for which no effective means has been available so far. The agent for inhibiting neurite elongation of the present invention is expected to be effective against post-zoster neuralgia, chronic pains and the like, for which no effective means has been available so far, because of the ability thereof to inhibit neurite elongation by inhibiting the action of BDNF. The antibody contained in the aforementioned inhibitor is capable of suppressing the activation of the autocrine system of BDNF by neutralizing the action of BDNF; furthermore, by this synergistic or additive suppressive effect, it is also possible to suppress the secondary reaction in which nerve cells are further induced to produce by the secreted BDNF. Furthermore, the prophylactic or therapeutic agent of the present invention is expected to ameliorate diseases with nerve hypersensitivity such as experience-dependent social aversion and stress via inhibition of the BNDF action.

### Brief Description of the Drawings

[FIG. 1A] FIG. 1A is a schematic diagram showing the positional relationship of fragments of the immediate-early (IE) 62 protein of varicella-zoster virus (VZV).
[FIG. 1B] FIG. 1B shows electrophoregrams of IE62 fragment-GST fusion proteins.
[FIG. 1C] FIG. 1C summarizes Western blotting that examined the reactivities of zoster or PHN patient sera to IE62 and BDNF.
[FIG. 1D] FIG. 1D shows examples of Western blots that examined the reactivities of zoster or PHN patient sera to IE62 and BDNF.
[FIG. 2A] FIG. 2A shows electrophoregrams showing the reactivities of anti-IE62 monoclonal antibody and anti-BDNF monoclonal antibody to IE62 fragments.
[FIG. 2B] FIG. 2B shows immunohistological staining of VZV-infected cells using anti-IE62 monoclonal antibody and anti-BDNF monoclonal antibody.
[FIG. 2C] FIG. 2C shows SDS-PAGE electrophoregrams of BDNF proteins and Western blot photographs using anti-IE62 monoclonal antibody and anti-BDNF monoclonal antibody.
[FIG. 3] FIG. 3 shows electrophoregrams that determined the epitopes of anti-IE62 monoclonal antibody and anti-BDNF monoclonal antibody.
[FIG. 4A] FIG. 4A shows that anti-IE62 monoclonal antibody increases the transcription of BDNF exons 3 to 5 and the transcription of Arc.
[FIG. 4B] FIG. 4B shows the morphology of dendrites of cortical (GABAergic) neurons cultured without (a) and with (b) the addition of IE62 and BDNF. The scale bar indicates 40 µm.
[FIG. 4C] FIG. 4C shows that anti-IE62 monoclonal antibody increases the area of nerve cell bodies.
[FIG. 4D] FIG. 4D shows that anti-IE62 monoclonal antibody increases the number of nerve branching points.
[FIG. 4E] FIG. 4E shows that anti-IE62 monoclonal antibody increases the total length of dendrites.
[FIG. 5A] FIG. 5A shows that anti-IE62 monoclonal antibody increases the area of spinal nerve cell bodies.
[FIG. 5B] FIG. 5B shows that anti-IE62 monoclonal antibody increases the number of spinal neuron branching points.
[FIG. 5C] FIG. 5C shows that anti-IE62 monoclonal antibody increases the total length of spinal neuron dendrites.
[FIG. 5D] FIG. 5D shows the morphology of the dendrites of dorsal nerve root neurons cultured without (a) (control) and with (b) the addition of IE62 and BDNF. The scale bar indicates 40 µm.
[FIG. 6A] FIG. 6A is a photograph of immunostaining of cerebral cortical cells using the monoclonal antibody KSG1. The magnification is x 1000.
[FIG. 6B] FIG. 6B is a photograph of immunostaining of mesencephalic cells using the monoclonal antibody KSG1. The magnification is x 1000.
[FIG. 6C] FIG. 6C is a photograph of immunostaining of cerebral cortical cells using the monoclonal antibody KSG4. The magnification is x 400.
[FIG. 6D] FIG. 6D is a photograph of immunostaining of mesencephalic cells using the monoclonal antibody KSG4. The magnification is x 1000.
[FIG. 7] FIG. 7 is a graph showing changes in the expression level of BDNF mRNA obtained with the addition of BDNF treated with PHN patient serum to fetal rat cerebral cortex in primary culture.

### Best Mode for Carrying out the Invention

The agent for promoting neurite elongation of the present invention comprises as an active ingredient an antibody that recognizes a varicella-zoster virus immediate-early protein and cross-reacts with a brain-derived neurotrophic factor. The antibody, when distinguished from the inhibitory antibody contained in the agent for inhibiting neurite elongation of the present invention described below, is referred to as the promoting antibody.

The aforementioned antibody is an antibody that recognizes a varicella-zoster virus immediate-early protein. Here, varicella-zoster virus (VZV) is a member of the genus Alphaherpesvirus, being the pathogenic medium for varicella and zoster. The VZV genome is a linear double-stranded DNA molecule; disclosed in, for example, J. Gen Virol. (1986), 67, 1759-1816 are the entire nucleotide sequence thereof and the amino acid sequence of the protein encoded thereby. Also disclosed as VZV (human herpesvirus 3) with Genbank Accession No: NC_001348 are the nucleotide sequence of the whole genome and the amino acid sequence of the protein encoded by the genome. The VZV genome encodes about 70 kinds of proteins. All genes of VZV are thought to be expressed in infected cells in the broad range of three dynamic classes: immediate-early (IE), early (E) and late (L). The VZV IE62 proteins are involved in the activation of the expression of all the three dynamic classes of the VZV proteins.

The aforementioned antibody is produced with an immediate-early protein, more preferably IE62, out of the proteins encoded by VZV, as an antigen. For IE62 protein as an antigen, disclosed in the aforementioned documents and Genbank and the like is the amino acid sequence thereof. For example, in the case of the human herpesvirus 3 VZV-Oka strain, the nucleotide sequence (SEQ ID NO:1) and amino acid sequence (SEQ ID NO:2) of IE62 are disclosed in Genbank Accession No:AY016449. For the human herpesvirus 3 VZV-wild strain (Kawaguchi strain), see the Journal of Virology 2002 p11447-144459.

The aforementioned promoting antibody preferably recognizes a peptide having the amino acid sequence at the 414-429 positions (PGYRSISGPDPRIRKT: SEQ ID NO:3) in the amino acid sequence of IE62 of SEQ ID NO:2. A promoting antibody that recognizes this peptide is preferable because the cross-reactivity with BDNF is more remarkable as defined below. The aforementioned peptide functions as an epitope for the promoting antibody in the present invention; as far as the function thereof as an antigenic determinant is not lost, about 1 to 3 amino acids may be deleted from the N-terminus and/or C-terminus of the amino acid sequence of SEQ ID NO:3. Alternatively, the aforementioned peptide, as far as the function thereof as an antigenic determinant is not lost, may have one to several (normally 1 to 5, preferably 1 to 3) additional amino acids at the N-terminus or C-terminus thereof. As the additional amino acid residues, cysteine residues introduced for the binding with a high molecular weight compound (protein) described below and the like can be mentioned.

The aforementioned antibody is capable of recognizing an immediate-early protein, preferably IE62, with high sensitivity and high specificity, and cross-reacts with a brain-derived neurotrophic factor (BDNF).

Regarding brain-derived neurotrophic factors (BDNF), disclosed in GenBank Accession No.NM_170731-170735 and NM_001709 are the nucleotide sequence and amino acid sequence of human BDNF. The BDNF with which the aforementioned promoting antibody cross-reacts is of the matured form, preferably a dimer of the matured form.

As mentioned herein, "antibodies" include natural type antibodies such as polyclonal antibodies and monoclonal antibodies, chimeric, humanized, and single-stranded antibodies produced using genetic recombination technology, human antibodies that can be produced using human antibody-producing transgenic animals and the like, antibodies prepared by phage display, and binding fragments thereof.

A binding fragment means a partial region of one of the above-described antibodies; and includes specifically, for example, F(ab')₂, Fab', Fab, Fv (variable fragment of antibody), sFv, dsFv (disulphide stabilized Fv), dAb (single domain antibody) and the like (Exp. Opin. Ther. Patents, Vol.6, No.5, p.441-456, 1996).

Any antibody class is acceptable; antibodies of any isotypes such as IgG, IgM, IgA, IgD and IgE are encompassed. Preferably, the class is IgG or IgM, and in view of the ease of purification and the like, IgG is more preferable.

A polyclonal antibody or a monoclonal antibody can be produced by a known ordinary method of production. Specifically, for example, an immunogen, along with Freund's Adjuvant as required, is given for immunization to a mammal, for example, a mouse, rat, hamster, guinea pig, rabbit, cat, dog, pig, goat, horse or bovine, preferably a mouse, rat, hamster, guinea pig, goat, horse or rabbit, in the case of a polyclonal antibody, or to a mouse, rat or hamster in the case of a monoclonal antibody.

In one embodiment, an IE62 peptide as an immunogen can be produced by a publicly known method. For example, a peptide comprising the amino acid sequence of SEQ ID NO:3 can be synthesized by a conventional method, allowed to form a complex with a high molecular weight compound (protein) such as bovine serum albumin (BSA), rabbit serum albumin (RSA), ovalbumin (OVA), keyhole limpet hemocyanin (KLH), thyroglobulin (TG), or immunoglobulin, and used as an immunogen.

For forming the above-described complex of an IE62 peptide and a high molecular weight compound and other purposes, one or several amino acids may be added. The number of amino acids added is not particularly limited, but taking into account the specificity of the antibody produced, the number is preferably 1 to 10, more preferably 1 to 5, still more preferably 1 to 2, and most preferably 1. Although the amino acids added may be located at any of the N-terminus and C-terminus of the polypeptide, the C-terminus is preferable.

The complex can be formed by a publicly known method being not particularly limited. For example, it is possible to react a carboxy group of the aforementioned IE62 peptide and a functional group of the aforementioned high molecular weight compound by the mixed acid anhydride method, the active ester method or the like to form a complex. Alternatively, it is also possible to introduce a cysteine residue into the C-terminus of the aforementioned IE62 peptide, and to bind the peptide with the aforementioned high molecular weight compound via the SH group being a side chain of the cysteine.

In another embodiment, for example, using an expression vector wherein a nucleotide that encodes a peptide comprising the amino acid sequence of SEQ ID NO:3 is joined to a nucleotide that encodes another protein (e.g., glutathione-S-transferase (GST)), a fusion protein with the other protein is expressed in a host by a conventional method and purified, and can be used as an immunogen.

Specifically, a polyclonal antibody can be produced as described below. An immunogen is injected to a mouse, rat, hamster, guinea pig, goat, horse or rabbit, preferably to a goat, horse or rabbit, more preferably to a rabbit, subcutaneously, intramuscularly, intravenously, into a footpad or intraperitoneally, once to several times, whereby the animal is immunologically sensitized. Normally, 1 to 5 immunizations are performed at intervals of about 1 to 14 days from initial immunization, and about 1 to 5 days after final immunization, a serum is acquired from the immunologically sensitized mammal.

Although a serum can be used as a polyclonal antibody, it is preferable that the antibody be isolated and/or purified by ultrafiltration, ammonium sulfate fractionation, euglobulin precipitation method, the caproic acid method, the caprylic acid method, ion exchange chromatography (DEAE or DE52 and the like), or affinity column chromatography using an anti-immunoglobulin column, a protein A/G column, an immunogen-crosslinked column or the like.

A monoclonal antibody is produced by preparing a hybridoma from a cell that produces the antibody, derived from the above-described immunologically sensitized animal, and a myeloma cell not having the capability of autoantibody production, cloning the hybridoma, and selecting a clone that produces a monoclonal antibody that exhibits specific affinity for the immunogen used to immunize the mammal and cross-reacts with BDNF.

A monoclonal antibody can be produced as described below. Specifically, an immunogen is injected once to several times, or transplanted, to a mouse, rat or hamster (including transgenic animals created to produce an antibody derived from another animal, like human antibody-producing transgenic mice) subcutaneously, intramuscularly, intravenously, in a footpad or intraperitoneally, whereby the animal is immunologically sensitized. Normally, 1 to 4 immunizations are performed at intervals of about 1 to 14 days from initial immunization, and about 1 to 5 days after final immunization, antibody-producing cells are acquired from the immunologically sensitized the mammal.

Preparation of a hybridoma (fusion cell) that secretes a monoclonal antibody can be performed according to the method of Kohler and Milstein et al. (Nature, Vol.256, p.495-497, 1975) or a modified method based thereon. Specifically, the hybridoma is prepared by cell-fusion of an antibody-producing cell contained in a spleen, lymph node, bone marrow, tonsil or the like, preferably in a spleen, acquired from a mammal immunologically sensitized as described above, and a myeloma cell not having the capability of autoantibody production, preferably derived from a mammal such as a mouse, rat, guinea pig, hamster, rabbit or human, more preferably from a mouse, rat or human.

Examples of useful myeloma cells for cell fusion include mouse-derived myeloma P3/X63-AG8.653 (653; ATCC No.CRL1580), P3/NSI/1-Ag4-1 (NS-1), P3/X63-Ag8.U1 (P3U1), SP2/0-Ag14 (Sp2/0, Sp2), PAI, F0 or BW5147, rat-derived myeloma 210RCY3-Ag.2.3., and human-derived myeloma U-266AR1, GM1500-6TG-A1-2, UC729-6, CEM-AGR, D1R11 or CEM-T15.

Screening for a hybridoma clone that produces a monoclonal antibody can be performed by culturing a hybridoma in, for example, a microtiter plate, measuring the reactivity of the culture supernatant of a well in which proliferation is observed to the immunogen used in the above-described immunological sensitization, and the reactivity of the supernatant to BDNF, by, for example, an enzyme immunoassay such as ELISA.

The hybridoma may be cultured using a medium (for example, DMEM containing 10% fetal bovine serum), and a centrifugal supernatant of the culture broth may be used as a monoclonal antibody solution. It is also possible to inject this hybridoma into the abdominal cavity of the animal from which it is derived to thereby produce ascites fluid, and to use the ascites fluid obtained as a monoclonal antibody solution. The monoclonal antibody, like the above-described polyclonal antibody, is preferably isolated and/or purified.

A chimeric antibody can be produced with reference to, for example, "Jikken Igaku (extra issue), Vol. 6, No.10, 1988", JP-B-HEI-3-73280 and the like; a humanized antibody can be prepared with reference to, for example, Japanese Patent Application Kohyo Publication No. HEI-4-506458, JP-A-SHO-62-296890 and the like; a human antibody can be prepared with reference to, for example, "Nature Genetics, Vol.15, p.146-156, 1997", "Nature Genetics, Vol.7, p.13-21, 1994", Japanese Patent Application Kohyo Publication No. HEI-4-504365, International Patent Application Publication WO94/25585, "Nikkei Science, June issue, pages 40 to 50, 1995", "Nature, Vol.368, p.856-859, 1994", Japanese Patent Application Kohyo Publication No. HEI-6-500233 and the like.

In preparing an antibody by phage display, an antibody such as Fab can easily be obtained from a phage library prepared for antibody screening by, for example, recovering and concentrating a phage with affinity for the antigen by biopanning. In this case, it is preferable that an antibody library be screened using a peptide having at least seven continuous amino acids selected from the amino acid sequence at the 414-429 positions of SEQ ID NO:2 as an antigen. For preferable antibody libraries and antibody screening methods, see the pamphlet for International Patent Application Publication No. 01/062907 and JP-A-2005-185281.
Present in the phage library are clones capable of reacting to all antigens. Therefore, with IE62 as the antigen, clones of anti-IE62 antibodies against all kinds of epitopes present in IE62 are obtained. If screening is performed on this set of clones using BDNF as the second antigen, cross-reacting antibodies are obtained. On the basis of the action on BDNF activity, an antibody that functions to suppress or promote human type BDNF activity can be selected. The human type antibody obtained is advantageous for human applications.

F(ab')₂ and Fab' can be produced by treating an immunoglobulin with the proteinase pepsin or papain, respectively. Fab can be produced by screening a Fab expression phage library in the same manner as the above-described method of antibody preparation by phage display.

The thus-obtained antibody that recognizes an IE62 protein makes it possible to detect or quantify the IE62 protein by a method based on an ordinary antigen-antibody reaction. The method is not particularly limited, and radioisotope immunoassay (RIA), enzyme immunoassay (e.g., ELISA), fluorescent or luminescent assay, agglutination method, immunoblot method, immunochromatography method and the like (Meth. Enzymol., 92, p.147-523 (1983), Antibodies Vol. II IRL Press Oxford (1989)) can be mentioned.

Being an active ingredient of the agent for promoting neurite elongation of the present invention, the aforementioned promoting antibody, unlike conventionally known antibodies against VZV, acts to promote neurite elongation via BDNF. Here, neurites are understood to include axons, dendritic processes and axon collaterals formed outside the nerve cells. Neurite elongation promotion refers to an increase in the total length of neurites as measured by examining the nerve cells under a microscope, compared with a control. In this case, a control refers to nerve cells kept under the same conditions except that the promoter of the present invention is not added.

The amount of antibody contained in the promoter of the present invention is not particularly limited, as far as the above-described action and effect are obtained, and the amount is normally 0.001 to 90% by weight, preferably 0.005 to 50% by weight, and more preferably 0.01 to 10% by weight.

The promoter of the present invention may comprise a carrier, in addition to the aforementioned antibody. As the carrier, carriers in common use in the field of pharmaceutical making can be used; examples include, but are not limited to, excipients such as sucrose, starch, mannitol, sorbitol, lactose, glucose, calcium phosphate and calcium carbonate; preservatives such as sodium benzoate, sodium hydrogen sulfite, methyl paraben and propyl paraben; stabilizers such as citric acid, sodium citrate and acetic acid; suspending agents such as methylcellulose, polyvinylpyrrolidone and aluminum stearate; dispersing agents such as surfactants; diluents such as water and physiological saline; base waxes such as glycerin and polyethylene glycol, and the like.

As cells on which the promoter of the present invention acts, nerve cells, particularly damaged nerve cells, and the like can be mentioned.

The promoter of the present invention can be used as an investigational reagent. Alternatively, the elongation of neurites can be promoted in vivo. For in vivo application, for example, a method wherein the promoter of the present invention is brought into contact with nerve cells in culture by being added to the medium, and the cultivation is continued as appropriate, can be mentioned.

The promoter of the present invention can be administered to a living organism to promote the elongation of neurites. The method of administration to a living organism is not limited; for example, subcutaneous injection, intramuscular injection, intraperitoneal injection, drip infusion and the like can be mentioned. In addition to systemic administrations such as oral administration, intravenous administration and intramuscular administration, topical administrations such as intracerebral, intramedullary (in spinal fluid), and unilateral dorsal hippocampal administrations can be mentioned. In topical administration into the brain, the promoter of the present invention can be contained in a carrier consisting of collagen.

The aforementioned promoting antibody that acts to promote neurite elongation is expected to regenerate nerve cells, particularly damaged nerve cells. The present invention provides a prophylactic or therapeutic agent for a nervous disease, comprising such antibody as an active ingredient.

The amount of promoting antibody contained in the prophylactic or therapeutic agent of the present invention is not particularly limited, as far as the above-described action and effect are obtained, and the amount is normally 0.001 to 90% by weight, preferably 0.005 to 50% by weight, and more preferably 0.01 to 10% by weight.

The prophylactic or therapeutic agent of the present invention may comprise a carrier, in addition to the aforementioned promoting antibody. As the carrier, those mentioned as examples for the aforementioned promoter can be mentioned.

Nervous diseases to which the prophylactic or therapeutic agent of the present invention is applicable are diseases whose pathologic condition is expected to be ameliorated by promoting neurite elongation. For example, diseases with damaged nerve cells caused by conditions such as cerebral stroke, anoxia, asphyxia, physical damage, exposure to toxins, malignant neoplasms, dementia, diabetes-related peripheral neuropathy, nerve degenerative disease (Parkinson's disease, Alzheimer's disease, Huntington's disease, amyotrophic lateral sclerosis, spinocerebellar degeneration and the like), immune nervous diseases (multiple sclerosis, Guillain-Barré syndrome, myasthenia gravis, myositis and the like), infectious diseases (encephalitis, meningitis and the like), angiopathies (cerebral infarction, cerebral hemorrhage and the like), other nervous diseases (myodystrophy, epilepsy, mitochondrial encephalomyopathy and the like) and the like, can be mentioned. Preferably, the prophylactic or therapeutic agent of the present invention is applied to anoxia, asphyxia, physical damage, exposure to toxins, malignant neoplasms, dementia, Alzheimer's disease, Parkinson's disease, and amyotrophic lateral sclerosis.

As the subject of administration of the promoter or prophylactic or therapeutic agent of the present invention, mammals such as mice, rats, hamsters, rabbits, cats, dogs, bovines, horses, sheep, monkeys, and humans can be mentioned.

Since the blood-brain barrier is locally destroyed in the onset of cerebral stroke, the delivery of the promoter of the present invention to the brain is expected to get facilitated. As blood-brain barrier passing agents, mannitol, arabinose, galactose, fructose, lactose, fructoligosaccharide, glucuronic acid, glycerol, sucrose, metrazol, etopside, synthetic bile salt, cholic acids, dimethyl sulfoxide, adenylic acids, inorganic salts or organic acids can be administered concurrently.

The promoter or prophylactic or therapeutic agent can be administered orally, intrarectally, parenterally, intracistemally, intravaginally, intraperitoneally, topically (in powders, ointments, gels, drip infusions, or transdermal patches and the like), or as intra-oral, oral or nasal sprays. The term "parenteral" as used herein refers to a mode of administration, including intravenous, intramuscular, intraperitoneal, intrasternal, subcutaneous and intra-articular injection and infusion.

The promoter or prophylactic or therapeutic agent can also be administered appropriately using a sustained release system. Examples of appropriate sustained-release agents encompass appropriate polymer substances (for example, semipermeable polymer matrixes in the form of moldings (for example, films or microcapsules)), appropriate hydrophobic substances (for example, as emulsions in oils of acceptable quality) or ion exchange resins, and poorly soluble derivatives (for example, poorly soluble salts).

The promoter or prophylactic or therapeutic agent of the present invention is formulated and administered in a regimen in compliance with the Good Medical Practice, in consideration of each patient's clinical condition, delivery site, method of administration, administration plan and other factors known to those skilled in the art. Therefore, "an effective amount" desired herein is determined with these considerations. As proposed generally, the pharmaceutically effective total amount of parenterally administered therapeutic agent per dose ranges from about 1 µg/kg/day to 10 mg/kg/day, based on the patient's body weight, and this relies on therapeutic considerations as stated above. More preferably, this dose is at least 0.01 mg/kg/day, and most preferably between about 0.01 mg/kg/day and about 1 mg/kg/day for a human. In the case of continuous administration, typically, the therapeutic agent is administered by injection at a dosing speed of about 1 µg/kg/hour to about 50 µg/kg/hour 1 to 4 times a day or by continuous subcutaneous infusion (for example, Mini Pump is used). A bag solution for intravenous administration can also be used. The length of treatment required for examination for changes and the interval from the treatment to the onset of a response varies depending on the desired effect.

The agent for inhibiting neurite elongation of the present invention comprises as an active ingredient an antibody that recognizes a varicella-zoster virus immediate-early protein and cross-reacts with a brain-derived neurotrophic factor (BDNF). The antibody contained as an active ingredient in the aforementioned inhibitor, unlike conventionally known antibodies against VZV, acts to inhibit neurite elongation by cross-reacting with BDNF and inhibiting the action of BDNF. This antibody is also simply referred to as the inhibitory antibody.

The aforementioned inhibitory antibody preferably recognizes a peptide having at least seven continuous amino acids selected from the amino acid sequence at the 268-341 positions (GPVEQLYHVLSDSVPAKGAKADLPFETDDTRPRKHDARGITPRVPGRSSGGKPRAFLALPGR SHAPDPIEDDSP: SEQ ID NO:18) in the amino acid sequence of IE62 shown by SEQ ID NO:2. An antibody that recognizes this peptide is preferable because it cross-reacts with BDNF and inhibits the action of BDNF. The aforementioned peptide functions as an epitope for the antibody in the present invention.

The aforementioned inhibitory antibody can be produced in the same manner as the method of producing the aforementioned promoting antibody, except that a peptide consisting of the amino acid sequence of SEQ ID NO:18 or a peptide having at least seven continuous amino acids selected from the amino acid sequence of SEQ ID NO:18 is used in place of a peptide consisting of the amino acid sequence of SEQ ID NO:3.

The amount of inhibitory antibody contained in the inhibitor of the present invention is not particularly limited, as far as the above-described action and effect are obtained, and the amount is normally 0.001 to 90% by weight, preferably 0.005 to 50% by weight, and more preferably 0.01 to 10% by weight.

The inhibitor of the present invention may comprise a carrier, in addition to the aforementioned inhibitory antibody. As the carrier, carriers in common use in the field of pharmaceuticals can be used; examples include, but are not limited to, excipients such as sucrose, starch, mannitol, sorbitol, lactose, glucose, calcium phosphate and calcium carbonate; preservatives such as sodium benzoate, sodium hydrogen sulfite, methyl paraben and propyl paraben; stabilizers such as citric acid, sodium citrate and acetic acid; suspending agents such as methylcellulose, polyvinylpyrrolidone and aluminum stearate; dispersing agents such as surfactants; diluents such as water and physiological saline; and base waxes such as glycerin and polyethylene glycol, and the like.

As cells on which the inhibitor of the present invention acts, nerve cells, particularly damaged nerve cells, and the like can be mentioned.

The inhibitor of the present invention can be used as an investigational reagent. Alternatively, the elongation of neurites can be inhibited in vivo. For in vivo application, for example, a method wherein the inhibitor of the present invention is brought into contact with nerve cells in culture by being added to the medium, and the cultivation is continued as appropriate, can be mentioned.

The inhibitor of the present invention can be administered to a living organism to inhibit the elongation of neurites. The method of administration to a living organism is not limited; for example, subcutaneous injection, intramuscular injection, intraperitoneal injection, drip infusions and the like can be mentioned. In addition to systemic administrations such as oral administration, intravenous administration and intramuscular administration, topical administrations such as intracerebral, intramedullary (in spinal fluid), and unilateral dorsal hippocampal administrations can be mentioned. In topical administration into the brain, the inhibitor of the present invention can be contained in a carrier consisting of collagen.

The aforementioned inhibitory antibody that acts to inhibit neurite elongation is expected to suppress a nerve hypersensitivity state. The present invention provides a prophylactic or therapeutic agent for a disease with nerve hypersensitivity, comprising such antibody as an active ingredient.

The amount of inhibitory antibody contained in the prophylactic or therapeutic agent of the present invention is not particularly limited, as far as the above-described action and effect are obtained, and the amount is normally 0.001 to 90% by weight, preferably 0.005 to 50% by weight, and more preferably 0.01 to 10% by weight.

The prophylactic or therapeutic agent of the present invention may comprise a carrier, in addition to the aforementioned inhibitory antibody. As the carrier, those mentioned as examples for the aforementioned inhibitor can be mentioned.

Diseases to which the inhibitor or prophylactic or therapeutic agent of the present invention is applicable are diseases whose pathologic condition is expected to get ameliorated by inhibiting neurite elongation. For example, post-zoster neuralgia, chronic pains, experience-dependent social aversion, stress and the like can be mentioned. Chronic pains are classified into:
(1) nociceptive pains, which are thought to occur as a result of persistent stimulation of nociceptors,
(2) neurogenic pains, which result from an abnormality in the function of nerve fibers involved in the mechanism for pain transmission or suppression, and
(3) psychogenic pains, in which affectional or emotional aspects are emphasized. As mentioned herein, chronic pains refer to two kinds: neurogenic pains (pains arising from nerve damage, compression and the like) and nociceptive pains (pains in cancers, rheumatism and the like). Experience-dependent social aversion (social frustration stress) refers to a state in which BDNF in the mesolimbic dopamine pathway plays an essential role.

As the subject of administration of the inhibitor or prophylactic or therapeutic agent of the present invention, mammals such as mice, rats, hamsters, rabbits, cats, dogs, bovines, horses, sheep, monkeys, and humans can be mentioned.

The inhibitor or prophylactic or therapeutic agent can be administered orally, intrarectally, parenterally, intracistemally, intravaginally, intraperitoneally, topically (in powders, ointments, gels, drip infusions, or transdermal patches and the like), or intraoral, oral or nasal sprays. The term "parenteral" as used herein refers to a mode of administration including intravenous, intramuscular, intraperitoneal, intrasternal, subcutaneous and intra-articular injection and infusion.

The inhibitor or prophylactic or therapeutic agent can also be administered appropriately using a sustained release system. Examples of appropriate sustained-release agents encompass appropriate polymer substances (for example, semipermeable polymer matrixes in the form of moldings (for example, films or microcapsules)), appropriate hydrophobic substances (for example, as emulsions in oils of acceptable quality) or ion exchange resins, and poorly soluble derivatives (for example, poorly soluble salts).

The inhibitor or prophylactic or therapeutic agent of the present invention is formulated and administered in a regimen in compliance with the Good Medical Practice, in consideration of each patient's clinical condition, delivery site, method of administration, administration plan and other factors known to those skilled in the art. Therefore, "an effective amount" desired herein is determined with these considerations. As proposed generally, the pharmaceutically effective total amount of parenterally administered therapeutic agent per dose ranges from about 1 µg/kg/day to 10 mg/kg/day, based on the patient's body weight, and this relies on therapeutic considerations as stated above. More preferably, this dose is at least 0.01 mg/kg/day, and most preferably between about 0.01 mg/kg/day and about 1 mg/kg/day for a human. In the case of continuous administration, typically, the therapeutic agent is administered by injection at a dosing speed of about 1 µg/kg/hour to about 50 µg/kg/hour 1 to 4 times a day or by continuous subcutaneous infusion (for example, Mini Pump is used). A bag solution for intravenous administration can also be used. The length of treatment required for examination for changes and the interval from the treatment to the onset of a response varies depending on the desired effect.

### Examples

The present invention is hereinafter described in detail by means of the following Examples, which, however, do not limit the scope of the invention in any way.

### Virus and cell culture

Oka strain of VZV (which is produced using virus derived from attenuated Varicella virus Oka strain (JP-B-SHO-53-41202 or US patent No. 3,985,615) as seeds, and practically used in countries worldwide (Requirements for Varicella Vaccine (Live) Adopted 1984; Revised 1993: WHO Technical Report Series, No.848, pp.22-38, 1994). The attenuated Oka strain was deposited with the ATCC as Deposition number of VR-795 on March 14, 1975.) and Kawaguchi strain of VZV (Journal of Virology, Nov. 2002, P.11447-11459) were propagated in human embryonic lung cells or a human lung cancer cell line A549 cells. The cells were grown and maintained in Eagle's minimum essential medium supplemented with 10 and 2% fetal bovine serum, respectively.

Primary cultures of rat cortical neurons were prepared from the cerebral cortexes of 17-18-day-old rat (Sprague-Dawley) embryos as described previously (Tabuchi, A., et al., J. Biol. Chem. 277, 35920-35931 (2002)). Briefly, small pieces of cerebral cortex were dissected by enzymatic (DNase I (Sigma) followed by trypsin (Sigma)) treatment and mechanical dissociation, and the cells were seeded at 5 × 10⁶ cells in a 60-mm culture dish (Iwaki). The cells were grown for 48 h in Dulbecco's modified Eagle's medium (Nissui) containing 10% fetal calf serum, and then the medium was replaced with serum-free Dulbecco's modified Eagle's medium (TIS medium) containing glucose (4.5 mg/ml), transferrin (5 µg/ml), insulin (5 µg/ml), sodium selenite (5 µg/ml), bovine serum albumin (1 mg/ml), and kanamycin sulfate (100 µg/ml). Cytosine arabinoside (Sigma) was also added at 2 µM to prevent the proliferation of glial cells. The medium was replaced with fresh TIS medium, but devoid of cytosine arabinoside, 2 h before DNA transfection.

### Example 1

### Expression of GST-IE62 fusion proteins and production of polyclonal antibodies

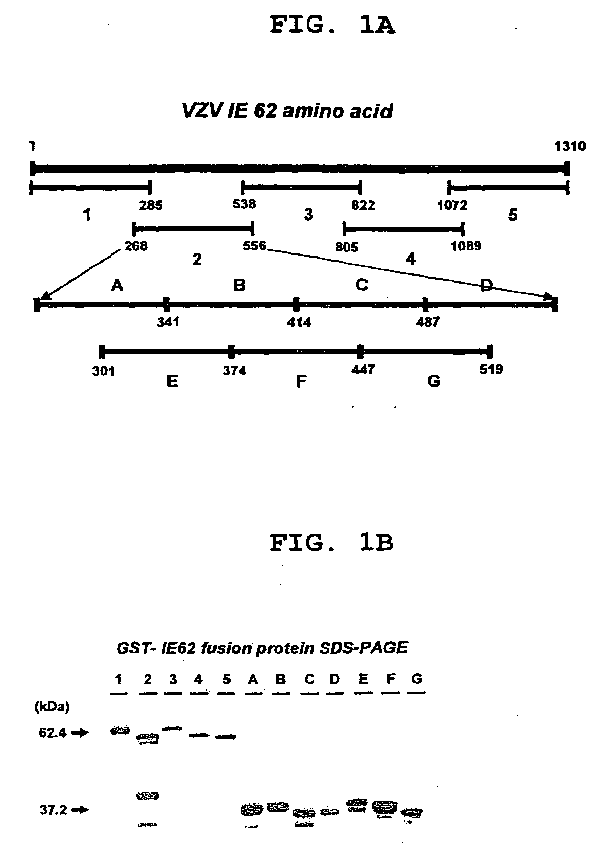

The partial VZV IE62 proteins were divided as fragments and synthesized as GST fusion proteins. The GST fusion proteins cover whole molecule with overlapping each other as shown in Fig. 1A. A recombinant plasmid expressing the glutathione-S-transferase (GST) - IE62 fusion protein was constructed by amplifying the IE62 gene in VZV gene and inserting the DNA fragment into vector pGEX-4T-1 (Pharmacia).

### 1) RNA Isolation and Reverse Transcription Polymerase Chain Reaction (RT-PCR)

Total RNA was extracted from the cultured cells using ISOGEN (NipponGene). RT-PCR was performed as described previously (Kawasaki, E. S., et al., Amplification of RNA. In PCR Protocol, A Guide to methods and applications, Academic Press, Inc., San Diego, 21-27 (1991)). Briefly, total RNA (1 µg) was reverse transcribed into cDNA in 20 µl of 1× first strand buffer. The buffer contained the followings: 0.5 µM oligo(dT)15 (5'-AAGCTTTTTTTTTTV-3') (SEQ ID NO:6) as a primer, 200 units of SuperScript II reverse transcriptase (Invitrogen), 400 µM dNTPs, and 10 units of RNase inhibitor (Invitrogen).

After reverse transcription, the reaction mixture was treated with 1.1 units of RNase H (Invitrogen) at 37°C for 20 min and used for PCR as cDNA solution. PCR was performed in 50 µl of 1× PCR buffer containing 1 µl of cDNA solution, 1.25 units of AmpliTaq Gold DNA polymerase (PerkinElmer Life Sciences), 1.5 mM MgCl₂, 200 µM dNTPs, and 0.5 µM primer pair. To distinguish four exons (exon I, exon II, exon III, and exon IV) of the rat BDNF gene, E-I (5'-ACTCAAAGGGAAACGTGTCTCT-3') (SEQ ID NO:7), E-II (5'-CGGTGTAGGCTGGAATAGACT-3') (SEQ ID NO:8), E-III (5'-CTCCGCCATGCA.ATTTCCACT-3') (SEQ ID NO:9), E-IV (5'-GTGACAACAATGTGACTCCACT-3') (SEQ ID NO:10), and E-Vas (5'-GCCTTCATGCAACCGAAGTA-3') (SEQ ID NO:11) were used.

For the internal control, glyceraldehyde-3-phosphate dehydrogenase (GAPDH) cDNA was amplified using GAPDH sense (5'-TCCATGACAACTTTGGCATTGTGG-3') (SEQ ID NO:12) and antisense (5'-GTTGCTGTTGAAGTCGCAGGAGAC-3') (SEQ ID NO:13) primers. For amplification of BDNF cDNA, the PCR conditions, after preheating at 95°C for 10 min, were as follows: denaturation at 94°C for 1 min, annealing at 55°C for 1 min, and extension at 72°C for 1.5 min for 32 (exon I), 31 (exon II), 26 (exon III), and 29 (exon IV) cycles, and a final extension at 72°C for 10 min. GAPDH amplification was carried out for 31 cycles under the same conditions. PCR products were separated by electrophoresis on 2% agarose gels, and the densities of the DNA bands stained with ethidium bromide were analyzed using a Bit-Map loader (ATTO) and software (NIH Image 1.52).

### 2) Expression of GST-IE62 fusion proteins

Constructed recombinant plasmid was transformed into Escherichia coli BL21 to obtain a transformant. The expression of a fusion protein was induced by culturing the transformant, adding 1mM IPTG (isopropyl-β-D-thiogalactopyranoside) thereto and incubating the transformant at 37°C or room temperature for 4hr or over night. The induced fusion protein was confirmed by SDS-PAGE (Fig. 1B). The induced fusion protein was purified by using a glutathione Sepharose 4B (Pharmacia) based on the manufacturer's instructions and its molecular weight identified by SDS-PAGE. The construction of recombinant plasmids was verified by their sequence. Verification of the antigenicity of the IE62 fusion proteins was performed by immunobloting procedures.

### 3) Production of polyclonal antibody

Fusion proteins 1 to 5 were immunized in rabbits according to the conventional method, and their immune sera were used for immunoblotting and the immunofluorescent antibody test to VZV-infected cells.

### Example 2

### Production of monoclonal antibody to IE62

Monoclonal antibody to IE 62 was produced using GST-IE62 fusion proteins essentially as described previously (Okuno et al, Virology 129, 357-368 (1983)). Hybridoma supernatant was screened by ELISA to GST protein and immunofluorescent antibody to VZV infected cells, and a hybridoma producing a monoclonal antibody to IE62 was subsequently cloned.

### Example 3

### Immunofluorescence antibody (IFA) assay

VZV-infected cells were fixed with acetone at -20°C and stained with monoclonal antibody to BDNF or IE62 and anti-mouse IgG serum conjugated with fluorescein as the first and second antibody, respectively. Antisera produced against IE62-GST fusion proteins in rabbits were confirmed by the immunofluorescent antibody to VZV-infected cells using anti-rabbit IgG serum conjugated with fluorescein (Jackson) as a second antibody.

### Example 4

### Western blotting

GST-IE62 fusion proteins or VZV-infected cell lysate were (was) loaded on the SDS gel, separated by electrophoresis and transferred to nitrocellulose membrane (Millipore). The membrane was probed by rabbit antisera to GST-IE62 fusion proteins, anti-IE62 monoclonal antibody (1:10,000 dilution; The Research Foundation for Microbial Diseases of Osaka University) or anti-human BDNF monoclonal antibody (0.5 µg/ml dilution; R&D Systems, Inc.). After further incubation with peroxidase-conjugated anti-rabbit IgG serum or goat anti mouse IgG serum for 1hr, the immunoblots were developed by the ECL method (Nacalai Tesque).

### Example 5

### Determination of epitope of IE62 recognized by anti-IE62 monoclonal antibody

The epitope of IE62 recognized by anti-IE62 monoclonal antibody was analyzed by the blocking of Western blot of IE62 with synthetic peptides. The epitope was overlapping area between fusion proteins C and F and corresponded to amino acids 414 to 447 of IE62. The area was divided into 3 parts, and three peptides (180 µg) were used as:
peptide I of PGYRSISGPDPRIRKT (SEQ ID NO:3),
peptide II of KRLAGEPGRQRQKSF (SEQ ID NO:4), and
peptide III of SLPRSRTPIIPPVSG (SEQ ID NO:5)
for blocking the interaction of anti-IE62 monoclonal antibody
to IE62 in Western blot.

### Example 6

### Effects of BDNF and anti-IE62 antibody to primary cultured neurons

Sprague-Dawley rats (postnatal day 0-1) were anesthetized with ketamine (50 mg/kg, i.p.), and then sacrificed by cervical dislocation. All efforts were made to minimize the number of animals used and their suffering. The experimental procedures met the regulations of the Animal Care Committee of Osaka University Graduate School of Medicine and the National Institutes of Health Guide for the Care and Use of Laboratory Animals. Solitary neurons were cultured by using conventional microisland methods (Kimura et al., J Neurophysiol. May; 77(5) 2805-2815, 1997). A piece of visual cortex was removed from the brain, enzymatically dissociated with papain (20 U/ml), and triturated with a fire-polished glass pipette. Neurons were plated on previously prepared glial microislands grown on collagen dots placed on an agarose sheet, and were grown in a solution based on Neurobasal A Medium (Gibco) supplemented with B27 (Gibco). Neurons thus cultured from the same animals were divided into two groups, and BDNF (recombinant proteins expressed in Escherichia coli: Sigma, recombinant proteins expressed in insect cells:R&D Systems) and anti-IE62 monoclonal antibody produced in Example 2 were added to the culture in a given concentration to obtain data from age-matched sister cultures. Recordings were carried out 10-15 days after plating.

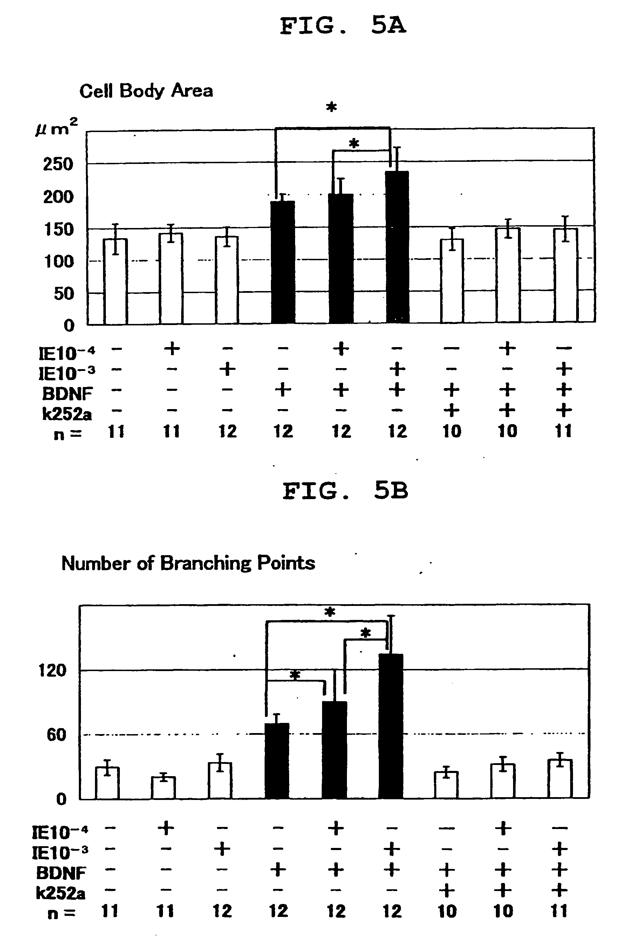

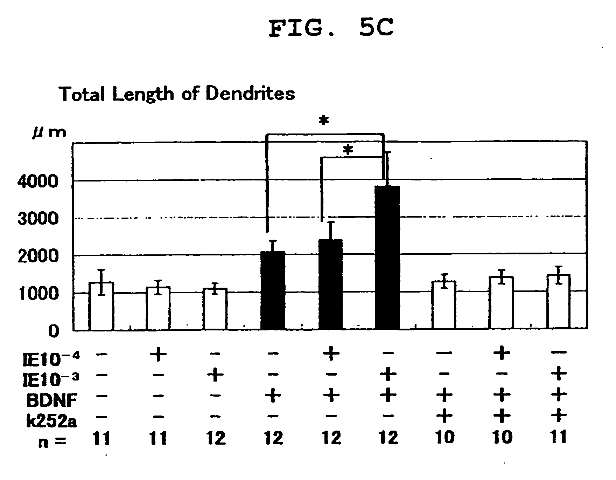

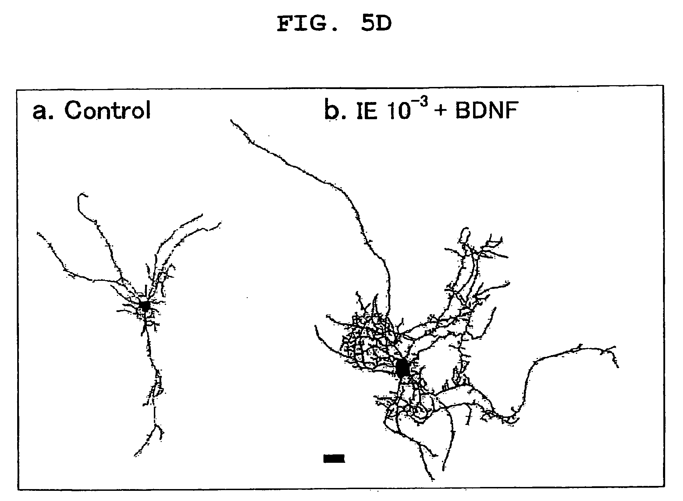

Next, effects of BDNF and anti-IE62 antibody as well as K252a, an inhibitor of Trk receptor tyrosine kinases to dorsal spinal nerve root neurons were examined. The results were shown in Fig. 5A-D.

### Example 7

### Analysis of morphology

After having recorded fluorescent images, neurons were incubated with anti-mouse IgG1 conjugated with biotin for 1 h at 37°C. Then ABC kit (Vector Laboratories) was used for visualization of MAP2. Neurolucida (MicroBrightField) attached on the inverted microscope (TE300; Nikon) was used for drawing of dendrites of neurons. The quantitative assessment of dendritic morphology was done with an analyzing software, Neuroexplore (MicroBrightField).

### Example 8

### Enhancement of transcription of Arc (Activity-regulated cytoskeleton-associated protein) and BDNF by anti-IE62 Monoclonal antibody

Total RNA was extracted from rat primary cultured neurons in Example 6 using ISOGEN (NIPPON GENE). Quantitative PCR was conducted as follows. Quantitative PCR amplification of rat BDNF gene exon III-V (Imamura L. et al., J Pharmacol., Exp. Ther 316: 136-143, 2006), Arc cDNA, and β-actin cDNA was carried out using Arc sense (5'-CGCTGGAAGAAGTCCATCAA-3') (SEQ ID NO:14) and Arc antisense (5'-GGGCTAACAGTGTAGTCGTA-3') (SEQ ID NO:15), and β-actin sense (5'-TTTGAGACCTTCAACACCCC-3') (SEQ ID NO:16) and β-actin antisense (5'-ACGATTTCCCTCTCAGCTGT-3') (SEQ ID NO:17) primers, respectively.

The thermal profile for PCR after predenaturation at 95°C for 10 min was as follows: denaturation at 95°C for 45 s, annealing at 55°C for 45 s and extension at 72°C for 1 min for 45 (exon I), 31 (exon II), 26 (exon III), and 29 (exon IV) cycles, and a final extension at 72°C for 10 min. An annealing temperature of 57°C was used for BDNF gene exon III-V amplification. Fluorescence was acquired at the end of each 72°C extension phase. The expression level of each mRNA was normalized using the level of β-actin mRNA.

### Results

### Fusion proteins

The constructs of recombinant plasmids were verified by their sequence. Immunogenicity of GST-IE62 fusion proteins 1 to 5 was verified by the Western blot with VZV antiserum. The antiserum positively stained the nuclei of VZV infected cells by IFA test and detected IE62 protein in Western blot. Then, IE62-GST fusion proteins were qualified and used for further characterization as IE62 proteins. Fig. 1A shows the schematic presentations of GST-IE62 fusion proteins 1 to 5 and A to G, comprising the whole IE62 molecule and their products.

### Recognition of IE62 and BDNF by zoster convalescent serum

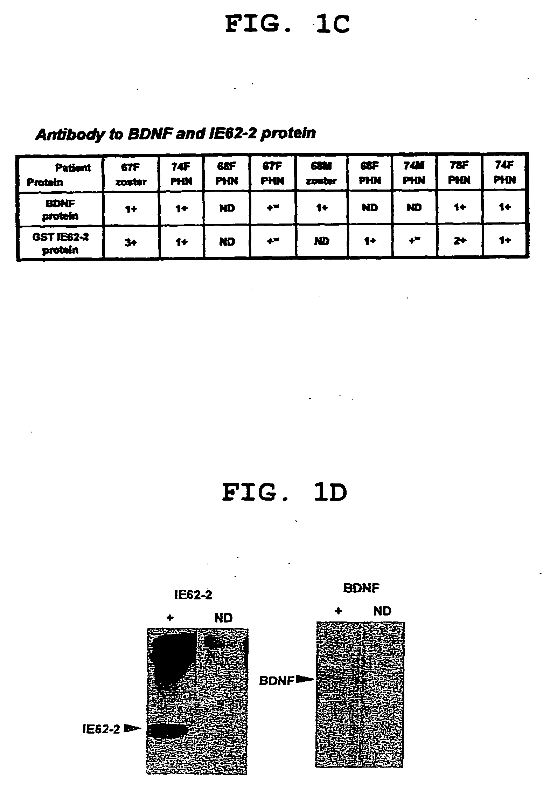

Fig. 1C and Fig. 1D show a summary of the reaction of sera from zoster patients with BDNF and GST-IE62 fusion protein 2 and one of representative pattern of Western blot. Serum of a patient (No.23) with zoster recognized the fragments 2 and F of IE62 and BDNF but that of a patient (No. 28) recognized the fragment 2 of IE62 but not the fragment F of IE62 nor BDNF by Western blot. Six sera reacted with BDNF and five of them recognized IE62 fusion protein(s). Two sera recognized IE62 but not BDNF. The recognition of IE62 and BDNF was not linked in all patients but sera from six patients suggested the association of antibody response to IE62 and BDNF.

### Cross recognition of BDNF and IE62 by their respective monoclonal antibodies

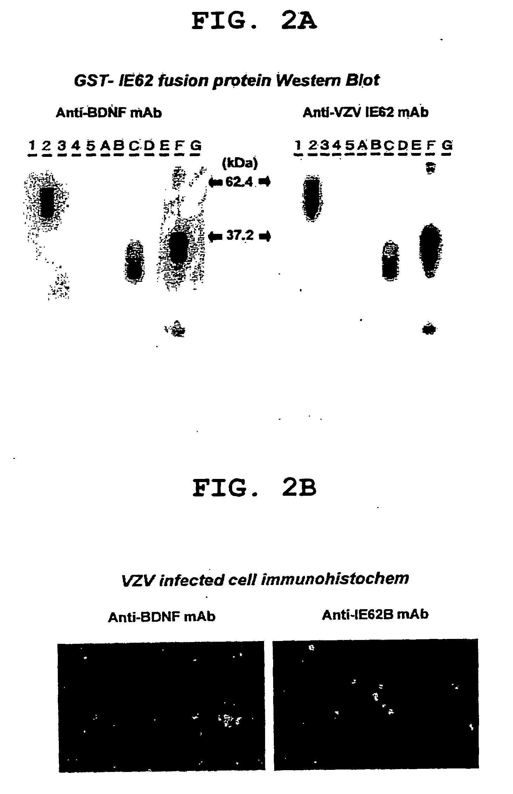

The present inventors produced monoclonal antibodies to IE62-GST fusion protein. The reactivity of these monoclonal antibodies to IE62-GST fusion proteins covered whole IE62 molecule as shown in Fig. 2A. Both anti-BDNF and anti-VZV IE62 monoclonal antibodies recognized the same regions of VZV IE62 in a similar manner as shown in Fig 2A. Fig. 2B shows IFA pattern stained by anti-BDNF and anti-VZV IE62 monoclonal antibodies and both mainly stained nuclei of infected cells. It was confirmed that anti-BDNF monoclonal antibody recognized the IE62 proteins by Western blot and IFA.

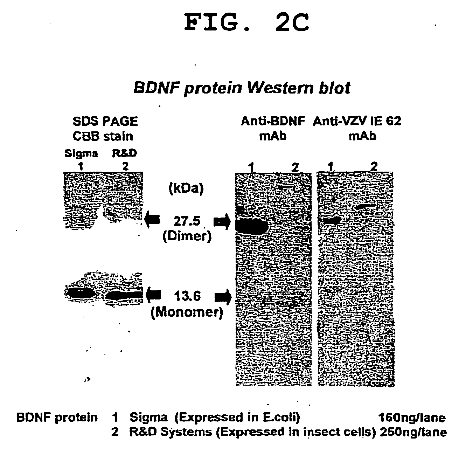

BDNF protein was blotted and probed by anti-VZV IE62 and anti-BDNF monoclonal antibodies and both recognized BDNF in a similar manner as shown in Fig. 2C. When BDNF was probed by anti-IE62 monoclonal antibody, it reacted with a dimer form of BDNF rather than its monomer form, indicating the recognition of conformational epitope created by BDNF dimer. Anti-BDNF monoclonal antibody which recognized IE62 reacted with the dimer form of BDNF rather than its monomer form in a similar manner to anti-IE62 monoclonal antibody. Thus immunological cross-reactivity of VZV IE62 and BDNF was confirmed by their respective monoclonal antibodies.

### Identification of the cross-reactive epitope of IE62 with BDNF

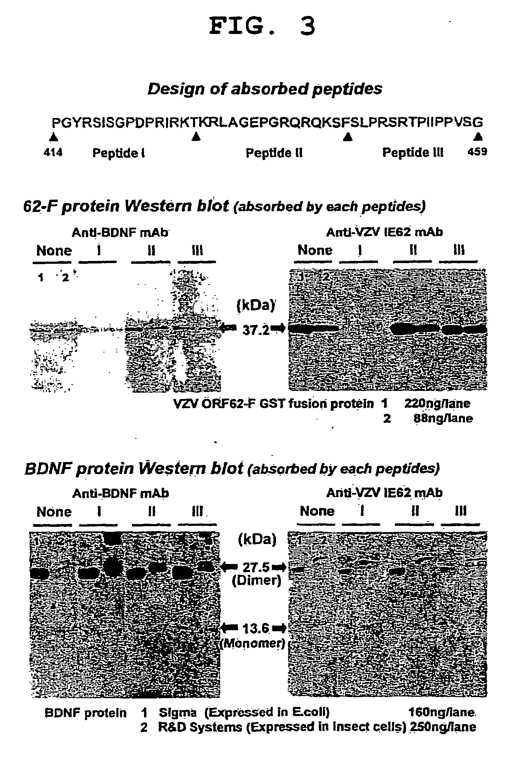

Anti-IE62 monoclonal antibody recognized GST-fusion protein 2 and further regions C and F (Fig. 2A). The overlapping regions of C and F suggested the amino acids 414 to 447 of IE62 protein and its three constituent peptides comprising the region were used to determine the epitope of VZV IE62 by blocking the reaction in Western blot. The peptide comprising amino acids 414 to 429 blocked the interaction of both anti-VZV IE62 antibody and anti-BDNF antibody, indicating the epitope of a peptide of amino acids 414 to 447 of IE62 was recognized by anti-VZV IE62 and anti-BDNF monoclonal antibody (Fig. 3). However this peptide failed to block the reaction of anti-IE62 and anti-BDNF monoclonal antibodies to BDNF in Western blot. Thus the epitopes of both monoclonal antibodies were a linear epitope of amino acids 414 to 447 of IE62 but the conformational epitope of BDNF created by the dimer form was not blocked in the same condition by either monoclonal antibody.

These results indicated cross-reacting epitope was amino acids 414 to 429 of VZV IE62.

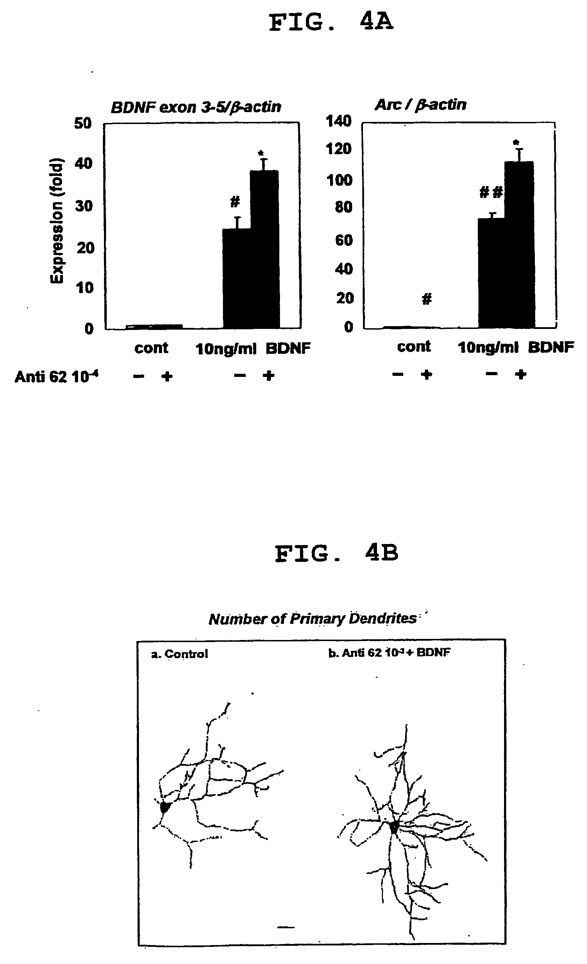

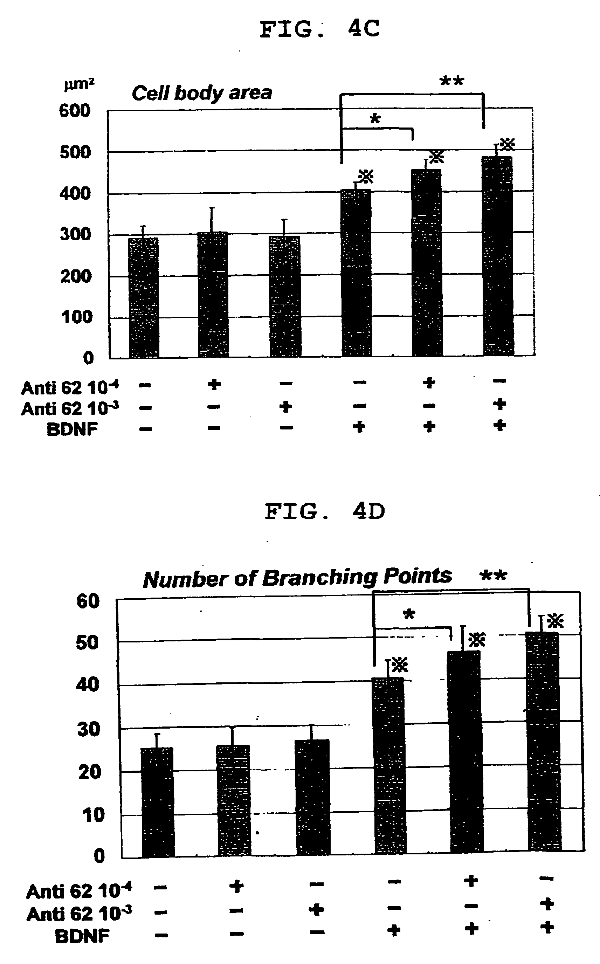

The present inventors characterized the effect of anti-VZV IE62 monoclonal antibody on the biological activity of BDNF with the cultured neurons. Anti-VZV IE62 monoclonal antibody significantly enhanced the transcription of Arc and BDNF exons 3-5 as shown in Fig. 4A. Further, anti-VZV IE62 monoclonal antibody significantly enhanced cell body area, the branching points, and the length of neuronal dendrites stimulated by BDNF (Fig. 4C to 4E). Thus anti-VZV IE62 monoclonal antibody did not neutralized but augmented the activity of BDNF in view of biological and biochemical markers. Thus two bioactive BDNF were connected by anti-VZV IE62 monoclonal antibody as a divalent BDNF and the divalent BDNF might bind two adjacent trkB molecules and transmit stronger signals than a single BDNF-trkB interaction, resulting in enhanced transcription of Arc and BDNF and development of dendrites of neuronal cells. Alternatively, binding of the antibodies to BDNF is considered to stabilize the confirmation of BDNF and to enhance the binding with TrkB receptor.

### Promotion of morphological development of GABAergic neurons by BDNF

To assess effects of BDNF on the inhibition of neuron development, the present inventors quantitatively analyzed the morphology of cortical (GABAergic) neurons which were confirmed to be immunoreactive to anti-GAD65 antibody. As shown in Fig. 4B(a) and (b), it is obvious that a cortical neuron cultured with anti-VZV IE62 antibody and BDNF had the larger cell body and much more abundant dendritic branches than those of another control neuron.

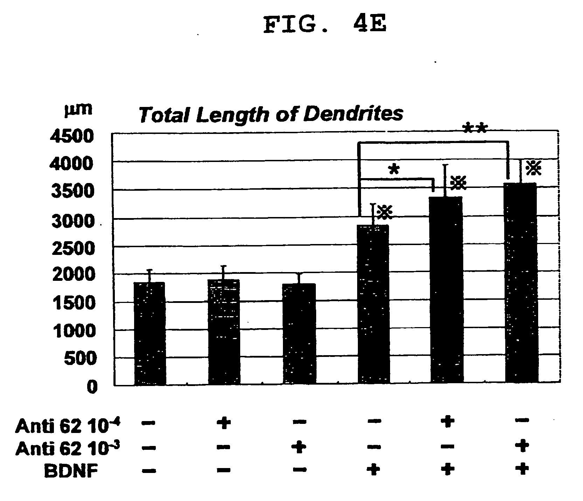

To quantify this finding, the present inventors calculated soma area and three parameters of dendritic morphology of each neuron (Fig. 4C-E). The mean area of soma of 10 neurons cultured with BDNF was significantly larger than that of 11 control neurons (P<0.01, ANOVA) (Fig. 4C). The mean number of primary dendrites of the BDNF-treated neurons was significantly larger than that of the control neurons (P<0.01, ANOVA). Also, the mean value of the total length of dendrites of the treated neurons was significantly larger than that of the control neurons (P<0.05, ANOVA) (Fig. 4E). Finally the mean number of dendritic branch points of the BDNF-treated neurons was significantly larger than that of the control neurons (P<0.01, ANOVA) (Fig. 4D). These results suggest that BDNF promotes development of soma and dendrites of cortical neurons.

### Promotion of morphological development of dorsal spinal nerve root neurons by BDNF

To confirm that such an effect of BDNF was elicited through an activation of TrkB receptors, we applied K252a, an inhibitor of Trk receptor tyrosine kinases, to a dorsal spinal nerve root neuron which was treated with BDNF. We found that the treatment of the agent blocked the proliferative action of BDNF on a dorsal spinal nerve root neuron. This was shown in the quantitative analysis of the soma area and three parameters of dendrites (Fig. 5A-D, a third column from right of each panel). The mean area of soma of 10 neurons treated with K252a was not significantly different from that of the control neurons. Also, the numbers of primary dendrites of the K252a-treated neurons were not significantly different from those of the control neurons. The total lengths of dendrites of the K252a-treated neurons were not significantly different from those of the control neurons. The mean number of dendritic branch points of the K252a-treated neurons was not significantly different from that of the control neurons.

It is clear that K252a suppresses both the action of BDNF and an enhancing action caused by IE62 antibody (IE10) (Fig. 5A-D, three columns from right of each panel). The dorsal spinal nerve root neuron is an excitatory neuron and directly transmits the pain. Therefore, it has been clarified that the antibody of the present invention promotes neurite elongation of an excitatory neuron of posterior horn of spinal cord via activation of BDNF.

### Example 9

### Preparation of antibodies that inhibit BDNF activity

### 1) Expression of CST IE62 fusion protein

A GST-IE62 fusion protein was prepared in the same manner as Example 1, except that a wild strain (Kawaguchi strain) of VZV, in place of the Oka strain of VZV, was used as an IE62 protein, and that a DNA that encodes the amino acid sequence at the 268-341 positions of SEQ ID NO:2 was amplified by PCR and joined with a DNA that encodes GST.

### 2) Preparation and screening of monoclonal antibodies

Monoclonal antibodies were produced using the GST-IE62 fusion protein obtained in 1) above, essentially in accordance with a method as described previously (Okuno et al, Virology 129, 357-368 (1983)). A hybridoma culture supernatant was screened by ELISA for GST protein and immunofluorescent antibody against VZV-infected cells, and hybridomas producing IE-62 monoclonal antibodies were cloned. Screening of the monoclonal antibodies obtained (KSG 1 to 13) with the inhibition of BDNF action as an index was performed by recording neurite elongation as described in Example 6, whereby the inhibitory antibody of the present invention was obtained.

### 3) Reactivities of the monoclonal antibodies and IE62 peptide

The reactivities of the monoclonal antibodies obtained (KSG 1 to 6, 8, 12 and 13) and the IE62 peptide (the fragment A or the fragment E in FIG. 1A) or GST protein were checked by Western blotting. None of the obtained monoclonal antibodies reacted to the GST protein. KSG 1, 2 and 6 reacted to the fragment A, and the other monoclonal antibodies reacted to the fragment A and the fragment E.

Next, the reactivities of monoclonal antibodies (KSG 1 to 8, 10, 12 and 13) with the IE62 peptide were checked by dot blot. Specifically, each of the GST fusion proteins with the IE62 peptide fragment A or fragment E was cleaved with trypsin, proteinase K, thrombin or V8 protease, and the reactivities of the cleaved products with the aforementioned monoclonal antibodies were checked by dot blot. As a result, it was estimated that the epitopes of KSG 1, 2 and 6 were present on the N-terminal side of the IE62 peptide fragment A, the epitopes of KSG 3, 4, 5, 12 and 13 were present in the center of the IE62 peptide fragment A and on the N-terminal side of the fragment E, and the epitope of KSG 8 was present at two positions: in the center of the IE62 peptide fragment A and on the N-terminal side of the fragment E, and on the C-terminal side thereof.

Next, two kinds of peptides:
peptide 1: GRSSGGKPRAFLALP (SEQ ID NO:19) and
peptide 2: DTRPRKHDARGITPR (SEQ ID NO:20), selected from SEQ ID NO:18
(GPVEQLYHVLSDSVPAKGAKADLPFETDDTRPRKHDARGITPRVPGRSSGGKPRAFLALPGR SHAPDPIEDDSP), were synthesized, the aforementioned monoclonal antibodies were absorbed with these peptides, and Western blotting was performed; KSG 8 was absorbed in the peptide 2, whereas KSG 3, 4, 5, 12 and 13 were unabsorbable in the peptides 1 and 2.

### 4) Nerve staining using antibodies that inhibit BDNF activity

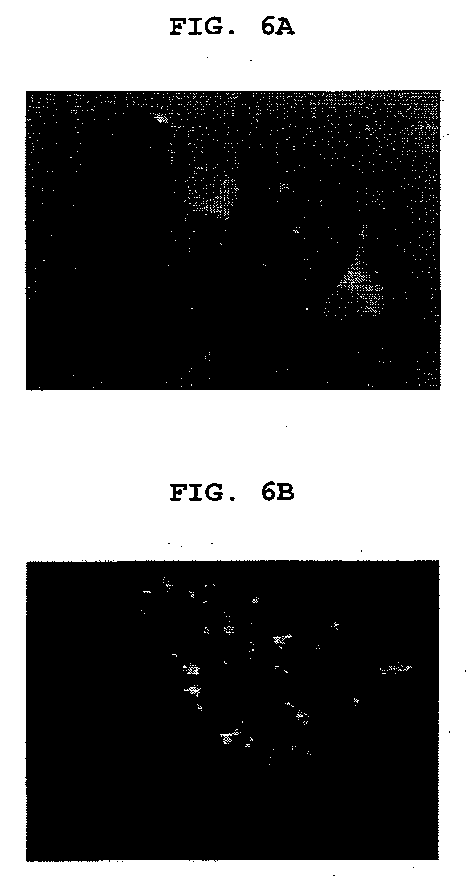

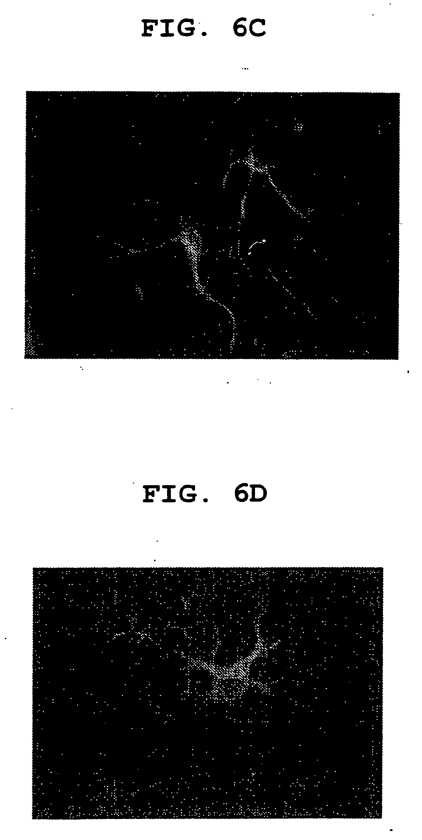

The cerebral cortex and mesencephalon were taken out from a fetal rat brain, subjected to primary culture, and immunologically stained using the monoclonal antibodies KSG 1 and 4. The results are shown in FIGS. 6A to 6D. The antibodies that inhibit BDNF activity were found to stain nerve cells.

### Example 10

### Changes in BDNF mRNA expression level with the addition of BDNF treated with PHN patient serum to fetal rat cerebral cortex in primary culture

Fetal rat cerebral cortex was subjected to primary culture, and changes in BDNF mRNA expression level with the addition of BDNF protein treated with PHN patient serum were examined. On day 17 of gestation, cerebral cortex was taken out from 10 fetal rats, treated with trypsin and DNase, and cultured in a poly-L-lysine-coated 6-well plate in the presence of Dulbecco's MEM containing 10% FCS. After cultivation for 3 days, the medium was exchanged with serum-free Dulbecco's medium, and the cerebral cortex was further cultured for 3 days. After a PHN patient serum (20 µl of 10-fold dilution) and BDNF protein, 10 ng (1 µl of 10 ng/µl), were reacted in ice for 4 hours, the reaction mixture was added to fetal rat cerebral cortical cells in primary culture, and the cells were further incubated for 3 hours. After the cells were twice washed with ice-cooled PBS, RNA was extracted. PolydT(15) and reverse transcriptase were added to the RNA obtained, and a cDNA was synthesized. The cDNA obtained was subjected to real time PCR with primers designed from the sequences of the exon 3 and exon 5 of BDNF and primers of β-actin as an internal standard, and the BDNF mRNA was quantified.

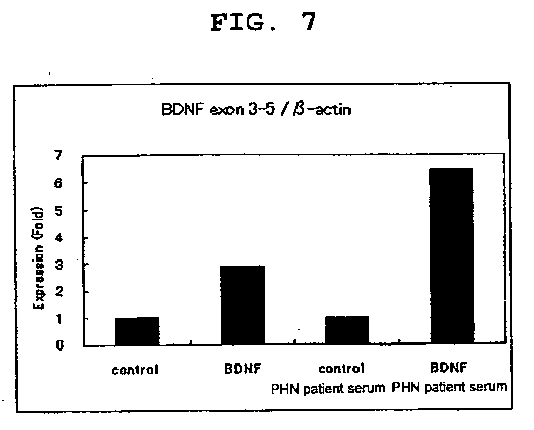

The results are shown in FIG. 7. From FIG. 7, it is seen that when the BDNF protein treated with a PHN patient serum was added to the fetal rat cerebral cortex, the BDNF mRNA expression level increased, compared with the use of the BDNF protein alone.

### Example 11

### Production of human type antibody

Since a site of IE62 that cross-reacts with BDNF immunologically has been identified, a human type antibody against an epitope thereof is screened using a human antibody library (AIMS4) and the IE62-GST fusion protein that expresses the site.

The IE62-GST fusion protein is coated over a test tube and reacted with the AIMS4 phage library; a phage that does not react is washed down, and a phage that reacted is allowed to proliferate with the addition of Escherichia coli. Next, the proliferating phage is repeatedly subjected to a process of selection by the panning method using a test tube coated with the IE62-GST fusion protein. The proliferating phage is selected using a BDNF-coated test tube and allowed to proliferate. The reactivities of the CP3-Fab produced by the selected phage to the IE62-GST fusion protein and BDNF are checked by Western blotting. An antibody clone exhibiting a reactivity similar to that of an anti-IE62 antibody that cross-reacts with BDNF is selected. The BDNF activity regulatory action of a Fab antibody is investigated using nerve cells in culture, and an IgG antibody is bound to a clone having activity. The regulatory actions of the Fab antibody and the IgG antibody are compared, and the appropriate one is used for experiment in anticipation of clinical applications.

### Example 12

### Effects on growth and development of cerebellar Purkinje's cell dendrites

An antibody of the present invention is inoculated to mice or rats at 1 day after birth; 3 weeks later, cerebellar Purkinje's cell dendrites are stained, and the effects on the growth and development of dendrites are investigated. Being cells uniformly distributed in the cerebellum, Purkinje's cells enable quantitation of dendrite elongation, and are suitable as a system for evaluating the effects on nerve cells.

From an evaluation of brain pathologic data on the mean length and branching points of dendrites after inoculation of the antibody obtained in Example 2, the antibody of Example 2 is judged to promote the growth of neurites. Therefore, the antibody leads to treatments of nervous diseases, such as nerve cell activation via BDNF, and promotion and regeneration of neural connections, and the like.

From an evaluation of brain pathologic data on the mean length and branching points of dendrites after inoculation of the inhibitory antibody obtained in Example 9, the antibody of Example 9 is judged to inhibit the growth of dendrites. It is evaluated, therefore, that 1) because the growth of dendrites via BDNF is inhibited, the excess neural connections considered to be formed in post-zoster neuralgia are restricted, and hence the onset of nociperception hypersensitivity can be suppressed, and that 2) in the case of chronic pains, likewise by inhibiting the formation of neural connections by the action of BDNF, the formation of chronic pains can be prevented.

### Example 13

### Recovery from memory disturbance, axon atrophy and synapse reduction with anti-IE62 monoclonal antibody in mouse model of Alzheimer's disease

For the purpose of functionally repairing neural circuit network collapse in dementia, screening for a suitable anti-IE62 monoclonal antibody is performed with neurite elongation promoting action as an index. Some IE62 monoclonal antibodies (e.g., IE62B) possess neurite elongation activity; for the purpose of determining whether or not a monoclonal antibody exhibiting neurite elongation activity acts on memory disturbance in dementia, there uses a mouse model of Alzheimer's disease generated by single-dose intraventricular administration of the active partial sequence 25-35 peptide of amyloid b (Ab), being the causal substance for Alzheimer's disease.

In mice receiving Ab (25-35), the acquirement and retention of space memory lessen significantly, compared with a control group. When brain sections are immunologically stained, significant reductions in axons and synapses are observed in the cerebral cortex and hippocampus.

In a group administered with an IE62 monoclonal antibody (e.g., IE62B) from 7 days after administration of Ab(25-35), it is confirmed that both the memory acquirement and retention capabilities are maintained at the levels observed in the control group, axons and synapses are kept at normal levels, and that dendrites are formed again.

Next, the actions of anti-IE62 monoclonal antibodies (e.g., IE62B) on rat cerebral cortical nerve cells in primary culture are investigated. The anti-IE62 monoclonal antibody specifically elongates the axons of cerebral cortical nerve cells. Even when the IE62 monoclonal antibodies are allowed to act on a state in which axon and dendrite atrophy are induced by treatment with Ab (25-35), the anti-IE62 monoclonal antibodies promote axon-specific elongation. From the results above, it is suggested that anti-IE62 monoclonal antibodies (e.g., IE62B) may suppress the axon atrophy and synapse reduction induced by Ab(25-35), and probably thereby induce a recovery from memory disturbance and be effective in reconstructing the neural circuit network.

### Example 14

### Investigation of preventive effect on cerebral stroke in stroke-prone spontaneously hypertensive rats (SHRSP)

Cerebral stroke preventive effect is investigated using stroke-prone spontaneously hypertensive rats (SHRSP). As major symptoms during cerebral stroke attacks in SHRSP, unilateral foreleg lifting, abnormal ataxia, hypersensitivity, decreased spontaneous activity, piloerection, body weight loss, decreased food consumption and the like are observed. Characteristic histological changes that have been identified include encephalomalacia, cerebral hemorrhage, vascular necrosis, cardiac hypertrophy, nephrosclerosis, mesenteric artery polyangiitis nodosa, testis atrophy and the like.

### (Methods)

Male SHRSP rats (10-week-old) are given 1% saline or tap water, received a specified dose of anti-IE62 monoclonal antibody (IE62B) administered intravenously, and examined. During the experimental period, food consumption, drinking water consumption, and body weight are measured once weekly, and the date of onset of cerebral stroke and the date of death are recorded. Blood pressure is measured by the tail cuff method using an automated sphygmomanometer for small animals (Ueda Seisakusho, UR-5000).

### (Results)

1. Effect of saline load:
   It is demonstrated that the 1% saline load promoted blood pressure elevation with almost no effect on body weight, and that the length of survival shortened to less than 1/2 compared with tap water rearing.
2. Preventing effect on cerebral stroke:
   The anti-IE62 monoclonal antibody (IE62B) extends the length of survival very remarkably, about 3.5 times compared with the saline load group, or by 100 days compared with the tap water rearing group; a remarkable effect is demonstrated.

### Example 15

### Nerve cell death suppressive action and regeneration promoting action of anti-IE62 antibody on Gerbil rat model of cerebrovascular disorder

Since the Gerbil rat is a rat free from the influence of the radial artery, anti-IE62 antibody is pre-administered, and the internal carotid artery is kept ligated for 5 minutes to cause angiopathy (destruction of the blood-brain barrier allows the antibody to penetrate the brain), and again opened. One week later, nerve cell death is investigated by Nissl's staining. The influence of this antibody is investigated with the drop of nerve cells in the hippocampal CA1 region, which is highly susceptible to ischemia and exhibits intense expression of BDNF, as an index.

### (Results)

It is confirmed that cell death in the CA1 region is ameliorated by the anti-IE62 antibody.

### Example 16

### Investigation of persistent expression of the BDNF and Arc genes in rat cerebral cortical nerve cells

### (Objective)

Brain-derived neurotrophic factor (BDNF) is important to the survival of nerve cells and the maintenance of the plasticity thereof, and Arc (Activity-regulated cytoskeleton-associated protein) is reportedly associated with synapse plasticity. The persistent expression control systems of the BDNF and Arc genes after administration of an IE62 monoclonal antibody (IE62B) to rats are investigated.

### (Methods)

E17 rat cerebral cortical nerve cells in primary culture are cultured at 2.5×10⁶ cells/35 mm dish. On day 6 of cultivation, BDNF is added; 3 hours later, the medium is exchanged (BDNF washout). BDNF and Arc mRNA expression levels are measured by quantitative RT-PCR.

### (Results and Discussion)

In the nerve cells in primary culture, the BDNF and Arc mRNA expression levels increases significantly from BDNF washout to 48 hours thereafter. As a result of a detailed investigation, it is demonstrated that the persistency of BDNF mRNA expression depends mainly on nerve activities.

Meanwhile, it is suggested that the persistency of Arc mRNA expression may depend on BDNF signals. Furthermore, BDNF increases the frequency of intracellular calcium oscillation, which is correlated with synapse density. From the results above, it is thought that the nerve activity-dependent and persistent expression control mechanism of the BDNF gene plays a basic role in maintaining the synapse function by adding persistency to the expression of the Arc gene.

### Example 17

### Investigation of the autonomic, persistent expression control system of BDNF in rat cerebral cortical nerve cells

### (Objective)

To demonstrate that the mRNA expression level of BDNF is persistently increased after administration of anti-IE62 monoclonal antibody, to analyze the mRNA expression level of Arc, which is reportedly associated with synapse plasticity, and to compare the result with that for BDNF mRNA expression.

### (Methods)

E17 rat cerebral cortical nerve cells in primary culture are cultured in a polyethylenimine-coated 35-mm dish at 2.5×10⁶ cells/2 mL. On day 6 of cultivation, BDNF (100 ng/mL) is added; 3 hours later, the medium is exchanged three times (BDNF washout). The BDNF mRNA and Arc mRNA expression levels are measured by a quantitative RT-PCR method.

### (Results and Discussion)

In the nerve cells in primary culture, both in the BDNF addition group and the BDNF washout group, compared with the control group, the BDNF mRNA expression level increases about 2 times persistently from 24 to 72 hours. In this case, the Arc mRNA expression level increases about 20 times, compared with the control group, in 24 hours, and about 2 times higher levels are maintained at 48 and 72 hours. These increases in mRNA are suppressed in the presence of the TrkB inhibitor K252a. When a BDNF-neutralizing antibody is used, the BDNF mRNA expression level does not change, but the Arc mRNA expression level decreases significantly.

Furthermore, the results obtained with the use of various inhibitors show that the BDNF mRNA and the Arc mRNA have different ways of signal transduction leading to the persistent expression thereof.

From the results above, it is suggested that the BDNF synthesized and secreted with the autonomic gene expression by BDNF may activate a plurality of signal transduction pathways via TrkB to thereby activate the expression of the BDNF and Arc genes persistently.

The BDNF-based autonomic/persistent expression control mechanism in nerve cells plays a basic role in maintaining nerve plasticity.

### Example 18

### Action of an antibody of the present invention in transgenic mice with the human APP gene introduced therein

Administration of anti-IE62 monoclonal antibody to PD-APP mice (Tg2576 mice), which strongly express the human-type APP gene, and which suffer from senile plaques in the brain with aging, prevents the formation of the senile plaques.

Mice receiving anti-IE62 monoclonal antibody (IE62B, KSG 1 to 13) restored the function of learning.

Anti-IE62 monoclonal antibody (IE62B, KSG 1 to 13) is once administered to Tg2576 mice. In the recipient mice, deposition of senile plaque amyloid decreases significantly, with no observed adverse reactions such as encephalitis. This method is expected to be applicable to humans as a prophylactic/therapeutic method for Alzheimer's disease.

An antibody of the present invention (anti-IE62 antibody that cross-reacts with BDNF) is injected to the above-described Tg2576 mice, and their memory after 1 month is investigated by a "water maze" experiment for memorization of shallows in a pool.

The mice receiving the antibody of the present invention restore the memory from the inability to memorize shallows before administration, to a level similar to that of wild-type mice.

### Industrial Applicability

According to the agent for promoting neurite elongation and prophylactic or therapeutic agent for a nervous disease of the present invention, it is possible to regenerate nerves after nerve damage, for which no effective means has been available so far. The agent for inhibiting neurite elongation of the present invention is also expected to be effective for post-zoster neuralgia, chronic pains and the like, for which no effective means has been available so far. Furthermore, according to the prophylactic or therapeutic agent of the present invention, amelioration is also expected for diseases with nerve hypersensitivity, such as experience-dependent social aversion and stress.

## Claims

1. An agent for use in a method of promoting neurite elongation comprising, as an active ingredient, an antibody that:
- recognizes varicella-zoster virus immediate-early 62 protein,
- recognizes a peptide having the amino acid sequence at the 414-429 positions of SEQ ID NO:2; and
- cross-reacts with a brain-derived neurotrophic factor.

2. The agent of claim 1, wherein the brain-derived neurotrophic factor with which the antibody cross-reacts is a dimer.

3. An agent for use in a method of preventing or treating a nervous disease, comprising, as an active ingredient, an antibody as defined in claim 1 or 2.

4. An antibody as defined in claim 1 or 2 for use in a method of preventing or treating a nervous disease.

5. The agent of claim 3 or the antibody of claim 4, wherein the nervous disease is a disease with nerve cells damaged by a condition selected from cerebral stroke, anoxia, asphyxia, physical damage, exposure to toxins, malignant neoplasms, dementia, Alzheimer's disease, Parkinson's disease and amyotrophic lateral sclerosis.

6. A commercial package comprising the agent of claim 1 or 2, and a written matter associated therewith, which states that the agent can or should be used for neurite elongation.

7. A commercial package comprising the agent of claim 3 or 5, and a written matter associated therewith, which states that the agent can or should be used for a nervous disease.

8. An agent for use in a method of inhibiting neurite elongation comprising, as an active ingredient, an antibody that:
- recognizes varicella-zoster virus immediate-early 62 protein,
- recognizes a peptide having at least seven continuous amino acids selected from the amino acid sequence at the 268-341 positions of SEQ ID NO:2, and
- cross-reacts with and inhibits a brain-derived neurotrophic factor.

9. An agent for use in a method of preventing or treating a disease with nerve hypersensitivity caused by a condition selected from post-zoster neuralgia, chronic pains, experience-dependent social aversion and stress, which agent comprises, as an active ingredient, an antibody as defined in claim 8.

10. An antibody as defined in claim 8 for use in a method of preventing or treating a disease with nerve hypersensitivity caused by a condition selected from post-zoster neuralgia, chronic pains, experience-dependent social aversion and stress.

11. A commercial package comprising the agent of claim 8, and a written matter associated therewith, which states that the agent can or should be used for inhibiting neurite elongation.

12. A commercial package comprising the agent of claim 9, and a written matter associated therewith, which states that the agent can or should be used for a disease with nerve hypersensitivity caused by a condition selected from post-zoster neuralgia, chronic pains, experience-dependent social aversion and stress.

13. A method of screening for an antibody as defined in claim 1 or 2, comprising the steps of:
- screening an antibody library using, as an antigen, a peptide having at least seven continuous amino acids selected from the amino acid sequence at the 414-429 positions of SEQ ID NO:2, and
- screening using a brain-derived neurotrophic factor as a second antigen.

14. A method of screening for an antibody as defined in claim 8, comprising the steps of:
- screening an antibody library using, as an antigen, a peptide having at least seven continuous amino acids selected from the amino acid sequence at the 268-341 positions of SEQ ID NO:2, and
- screening using a brain-derived neurotrophic factor as a second antigen.

15. The method of claim 13 or 14, wherein the antibody library is a human antibody library.

## Patentansprüche

1. Mittel zur Verwendung bei einem Verfahren zur Förderung der Neuritenverlängerung, das als aktiven Bestandteil einen Antikörper umfasst, der:
- das unmittelbar-früh exprimierte 62-Protein des Varicella-zoster-Virus erkennt;
- ein Peptid erkennt, das die Aminosäuresequenz auf den Positionen 414-429 von SEQ ID Nr. 2 aufweist; und
- mit dem vom Gehirn stammenden neurotrophen Faktor kreuzreagiert.

2. Mittel gemäß Anspruch 1, wobei der vom Gehirn stammende neurotrophe Faktor, mit dem der Antikörper kreuzreagiert, ein Dimer ist.

3. Mittel zur Verwendung bei einem Verfahren zur Prävention oder Behandlung einer Nervenkrankheit, das als aktiven Bestandteil einen Antikörper umfasst, wie er in Anspruch 1 oder 2 definiert ist.

4. Antikörper, wie er in Anspruch 1 oder 2 definiert ist, zur Verwendung bei einem Verfahren zur Prävention oder Behandlung einer Nervenkrankheit.

5. Mittel gemäß Anspruch 3 oder Antikörper gemäß Anspruch 4, wobei die Nervenkrankheit eine Krankheit ist, bei der Nervenzellen durch einen Zustand geschädigt sind, der aus einem Hirnschlag, Anoxie, Asphyxie, physischer Schädigung, Einwirkung von Toxinen, malignen Neoplasmen, Demenz, Alzheimer-Krankheit, Parkinson-Krankheit und amyotropher Lateralsklerose ausgewählt ist.

6. Kommerzielle Packung, die das Mittel gemäß Anspruch 1 oder 2 und ein damit verbundenes Schriftstück, welches besagt, dass das Mittel zur Neuritenverlängerung verwendet werden kann oder sollte, umfasst.

7. Kommerzielle Packung, die das Mittel gemäß Anspruch 3 oder 5 und ein damit verbundenes Schriftstück, welches besagt, dass das Mittel für eine Nervenkrankheit verwendet werden kann oder sollte, umfasst.

8. Mittel zur Verwendung bei einem Verfahren zur Hemmung der Neuritenverlängerung, das als aktiven Bestandteil einen Antikörper umfasst, der:
- das unmittelbar-früh exprimierte 62-Protein des Varicella-zoster-Virus erkennt;
- ein Peptid erkennt, das wenigstens sieben aufeinanderfolgende Aminosäuren aufweist, die aus der Aminosäuresequenz auf den Positionen 268-341 von SEQ ID Nr. 2 ausgewählt sind; und
- mit dem vom Gehirn stammenden neurotrophen Faktor kreuzreagiert und diesen dabei hemmt.

9. Mittel zur Verwendung bei einem Verfahren zur Prävention oder Behandlung einer Krankheit mit Nervenüberempfindlichkeit, die durch einen Zustand verursacht ist, der aus postzosterischer Neuralgie, chronischen Schmerzen, erfahrungsabhängiger sozialer Aversion und Stress ausgewählt ist, wobei das Mittel als aktiven Bestandteil einen Antikörper umfasst, wie er in Anspruch 8 definiert ist.

10. Antikörper, wie er in Anspruch 8 definiert ist, zur Verwendung bei einem Verfahren zur Prävention oder Behandlung einer Krankheit mit Nervenüberempfindlichkeit, die durch einen Zustand verursacht ist, der aus postzosterischer Neuralgie, chronischen Schmerzen, erfahrungsabhängiger sozialer Aversion und Stress ausgewählt ist.

11. Kommerzielle Packung, die das Mittel gemäß Anspruch 8 und ein damit verbundenes Schriftstück, welches besagt, dass das Mittel zur Hemmung der Neuritenverlängerung verwendet werden kann oder sollte, umfasst.

12. Kommerzielle Packung, die das Mittel gemäß Anspruch 9 und ein damit verbundenes Schriftstück, welches besagt, dass das Mittel für eine Krankheit mit Nervenüberempfindlichkeit, die durch einen Zustand verursacht ist, der aus postzosterischer Neuralgie, chronischen Schmerzen, erfahrungsabhängiger sozialer Aversion und Stress ausgewählt ist, verwendet werden kann oder sollte, umfasst.

13. Verfahren zum Screening nach einem Antikörper, wie er in Anspruch 1 oder 2 definiert ist, umfassend die Schritte:
- Durchmustern einer Antikörperbibliothek, wobei als Antigen ein Peptid verwendet wird, das wenigstens sieben aufeinanderfolgende Aminosäuren aufweist, die aus der Aminosäuresequenz auf den Positionen 414-429 von SEQ ID Nr. 2 ausgewählt sind; und
- Durchmustern unter Verwendung eines vom Gehirn stammenden neurotrophen Faktors als zweites Antigen.

14. Verfahren zum Screening nach einem Antikörper, wie er in Anspruch 8 definiert ist, umfassend die Schritte:
- Durchmustern einer Antikörperbibliothek, wobei als Antigen ein Peptid verwendet wird, das wenigstens sieben aufeinanderfolgende Aminosäuren aufweist, die aus der Aminosäuresequenz auf den Positionen 268-341 von SEQ ID Nr. 2 ausgewählt sind; und
- Durchmustern unter Verwendung eines vom Gehirn stammenden neurotrophen Faktors als zweites Antigen.

15. Verfahren gemäß Anspruch 13 oder 14, wobei die Antikörperbibliothek eine Human-Antikörperbibliothek ist.

## Revendications

1. Agent pour une utilisation dans un procédé d'activation de l'élongation des neurites comprenant, en tant que principe actif, un anticorps qui :
- reconnaît la protéine immédiate précoce 62 du virus varicelle-zona,
- reconnaît un peptide ayant la séquence d'acides aminés au niveau des positions 414 à 429 de SEQ ID NO : 2 ; et
- présente une réactivité croisée avec le facteur neurotrophique d'origine cérébrale.

2. Agent selon la revendication 1, dans lequel le facteur neurotrophique d'origine cérébrale avec lequel l'anticorps présente une réactivité croisée est un dimère.

3. Agent pour une utilisation dans un procédé de prévention ou de traitement d'une maladie nerveuse, comprenant, en tant que principe actif, un anticorps tel que défini dans la revendication 1 ou 2.

4. Anticorps tel que défini dans la revendication 1 ou 2, pour une utilisation dans un procédé de prévention ou de traitement d'une maladie nerveuse.

5. Agent selon la revendication 3 ou anticorps selon la revendication 4, où la maladie nerveuse est une maladie avec des cellules nerveuses endommagées par une affection choisie parmi un accident vasculaire cérébral, une anoxie, une asphyxie, un dommage physique, une exposition à des toxines, des néoplasmes malins, une démence, la maladie d'Alzheimer, la maladie de Parkinson et la sclérose latérale amyotrophique.

6. Conditionnement commercial comprenant l'agent selon la revendication 1 ou 2, et une notice qui y est associée, qui déclare que l'agent peut ou devra être utilisé pour l'élongation des neurites.

7. Conditionnement commercial comprenant l'agent selon la revendication 3 ou 5, et une notice qui y est associée, qui déclare que l'agent peut ou devra être utilisé pour une maladie nerveuse.

8. Agent pour une utilisation dans un procédé d'inhibition de l'élongation des neurites comprenant, en tant que principe actif, un anticorps qui :
- reconnaît la protéine immédiate précoce 62 du virus varicelle-zona,
- reconnaît un peptide ayant au moins sept acides aminés consécutifs choisis dans la séquence d'acides aminés au niveau des positions 268 à 341 de SEQ ID NO : 2, et
- présente une réactivité croisée avec et inhibe un facteur neurotrophique d'origine cérébrale.

9. Agent pour une utilisation dans un procédé de prévention ou de traitement d'une maladie présentant une hypersensibilité nerveuse provoquée par une affection choisie parmi une névralgie post-zona, des douleurs chroniques, une aversion sociale dépendante de l'expérience et un stress, lequel agent comprend, en tant que principe actif, un anticorps tel que défini dans la revendication 8.

10. Anticorps tel que défini dans la revendication 8 pour une utilisation dans un procédé de prévention ou de traitement d'une maladie présentant une hypersensibilité nerveuse provoquée par une affection choisie parmi une névralgie post-zona, des douleurs chroniques, une aversion sociale dépendante de l'expérience et un stress.

11. Conditionnement commercial comprenant l'agent selon la revendication 8, et une notice qui y est associée, qui déclare que l'agent peut ou devra être utilisé pour l'inhibition de l'élongation des neurites.

12. Conditionnement commercial comprenant l'agent selon la revendication 9, et une notice qui y est associée, qui déclare que l'agent peut ou devra être utilisé pour une maladie présentant une hypersensibilité nerveuse provoquée par une affection choisie parmi une névralgie post-zona, des douleurs chroniques, une aversion sociale dépendante de l'expérience et un stress.

13. Procédé de criblage à la recherche d'un anticorps tel que défini dans la revendication 1 ou 2, comprenant les étapes suivantes :
- le criblage d'une banque d'anticorps en utilisant, comme antigène, un peptide ayant au moins sept acides aminés consécutifs choisis dans la séquence d'acides aminés au niveau des positions 414 à 429 de SEQ ID NO : 2, et
- le criblage en utilisant un facteur neurotrophique d'origine cérébrale comme second antigène.

14. Procédé de criblage à la recherche d'un anticorps tel que défini dans la revendication 8, comprenant les étapes suivantes :
- le criblage d'une banque d'anticorps en utilisant, comme antigène, un peptide ayant au moins sept acides aminés consécutifs choisis dans la séquence d'acides aminés au niveau des positions 268 à 341 de SEQ ID NO : 2, et
- le criblage en utilisant un facteur neurotrophique d'origine cérébrale comme second antigène.

15. Procédé selon la revendication 13 ou 14, dans lequel la banque d'anticorps est une banque d'anticorps humains.


### SEQUENCE LISTING

<110> The Research Foundation f or Mcrobial Diseases of Osaka
   University
   RIKEN
<120> An agent f or promoting Neurite elongation and agent f or supr essi ng Neurite elongation
<130> 091172
<150> JP 2006/312236
   <151> 2006- 11 - 17
<160> 20
<170> Patent Iversion 3.3
<210> 1
   <211> 4490
   <212> DNA
   <213> Varicell azoster
<400> 1
   tcacccccga ctctgcgggg ggctcctccc cccgcgccct ccccacacat cgtcctcgtc    60
   ctcggaggac gaggacgagg acaacagctc caccttgacc gccgggcgca agcccacccg   120
   gcggtctcgc agcacacccg gggccaccga cacgatgctc accccaaagg atgaccccgg   180
   tgcgtccccg tcgtccccgc ccccctcctc gctgtcccac gcgtcttcac accccacctc   240
   ccaatcgtcc ggctccaaag cgtgttctct gtcgtctgcg gtgcgccgct gtcgccccgc   300
   ctgggtttct gccggccgtt ccgagccccc gtggtgtccg aacacgaacc gtgttccgtc   360
   gctcccctcc aacaccgtct ccgcggcccc aaaaccgggc ggccacatta ctctgggaat   420
   cggggggagg gcattccgag cctcgtccgc cgacgcatac agcgccaccg accgaccggc   480
   cacgggtgga agcacgagtg gttccgcggc agggtcgggt tccagcaggg cgtggcggca   540
   aaacaccctc gcccaggtgg gtacgtcgcc ggcctccggc ccggcggccc ccggtctccg   600
   tccctcggga aggaagacgg gtcgaagcgc ggcacccagg ccccatcggt ttgctgcgcg   660
   gtggctatgt gccgcctcgt ccacaaagtc ggctgccccg agccccagac cccgagactg   720
   tcgcgcgagg tccttgcaac cgtcaaaacc cggcagcacg tactgccggt attcacgggg   780
   cgacaggggg acgcgggtct tggggcccgc gcgggtacac acggtgtatg cgacgttccc   840
   accgcggcac aaacacaggg gttgttcgcc cgggtacagg ttggcaaacg cagtctcgat   900
   acgagcaaaa ctcgctggcc caaaggtgcg cgacgatgca aacacggccc gggcgagtcc   960
   ttctgtgacc gccgagtctg gccatcggac gacggcctgg gcgtccggtc gcgccggggc  1020
   ccggacgtac acgtgatact gagacaaagc gggtccatcc ccgggccacc tctcgagggc  1080
   caccgcgtcc aacaccagca accggcgccg ggcagaggcc aaccgcgagc ctagatactc  1140
   gacggccccg gcaaaggcca ggtctcgggt cgacagtaat aaaacgcccc gggcgttcaa  1200
   agcggacacg tccggcgggc cggtccagtt cccggcccag gcatgagtgc tcggcaggca  1260
   caaccggtta ctcagggctg ccaggaccac agacagtccc cctcgggatg gactccatga  1320
   cggtcccgga tctgtcgcga gggtgctctc gagggggccg ttgatgtcct ctccgggcaa  1380
   cggatcgtag atgatcagaa gcctcacatc ctccgggtct gggatctgcc gcatccaggc 1440
   gcacctccgt cgcagcgcct ccactccgct gggtggacca aaccgtcggt ctcctccgcc 1500
   cggacgccga gcggcgattt ccgccaaggc gccgggatca aagcttagcg cagggcgcca 1560
   ggccgtggga aacaatgggt cgtcgaccag acgggcgatg gtttcggggg tacagtacgc 1620
   cttgcgagcc tggtccgacg ggaccggggt atgcagggcc ccccggggaa tacgccgaaa 1680
   tcccccgttt ggggccggtc cgtcaagtgg catcgttatt acggcggggg gatccaccac 1740
   agggcccgag gtgatggtca cgggctcgga tacccgcctc ttggccttgg aaaccacatg 1800
   atcgtctgca acccgggcgt ccgcgacggg tgtctcccta atcttgtcga ggaggcttct 1860
   gctctcgact ggctgggact tgcgcttgcg cggagttcgt aaacgatcat ccggtggaca 1920
   cacagaaaga gagcgtgcgg cggccgacgg ctgagggtcg ggagtctgtg tggccggggt 1980
   tgttggagaa gggtgaccgc gggagatccg cgccgccgga ctggagcccg ttgcctcggg 2040
   gtatgccatg ccggcaaagg ctctgcggag actctgtagg ataaagtgtt tttgggcccg 2100
   gtcgtaccga cggctcatag ccacggccgc ggccgcgtgg gggagagccc agagggcctc 2160
   ccccgtggcc atggcttcgc ctacatgcgg aacgggagac gctacgctcc ccgtaacggc 2220
   ggtacccgcc cgtcccggtg gcaacagctt ttggtagaac tggttcaggg ccgagttgac 2280
   accggtcagc ttggggttct ggagccatgc tatagggtct ctgtctggac agtagatcag 2340
   gttaatcagc gcgcggtact gtctagccgg atctcccaac tccggcacgt aaagcggcac 2400
   gggttcagtt gaggcctcgt aacgagcccg cgccgctctc acagcctcat cctcccagtg 2460
   accctctctg gtctccccgg acggtccaaa ccgcaccctg ttggatggga ggggcgccga 2520
   tccgggccaa gggcttccgt cgggcatcat gagcggcccc gacaccgggg gaattatcgg 2580
   ggttctggat cgcggcaggg aaaatgattt ctgtctctgg cgccccggtt cccccgcaag 2640
   acgtttggtc ttacgaatcc tcggatcggg accgctgatg gatcgatatc ccggttggat 2700
   attttgtttc gtcgacccac catcatttga gtccgaatca tccgaatttg acggggaagg 2760
   ggcgtgttcg cgtccggacc tgctgcctgt agtttcactt cccaccgaaa cgcgccgggg 2820
   ttcatcgtct tcatcctccg atgacgatcc ccacgacgag gaagaggatg aagacgaaac 2880
   aaactcacga ctctttggct ttttctccac cgggctgtca tcctcaatcg ggtctggtgc 2940
   gtgggatctt cccggcaggg ccaaaaacgc tctaggtttg ccccccgacg aacgtccagg 3000
   gacgcgaggt gttatacccc gggcatcatg tttccttggg cgggtatcat cggtctcaaa 3060
   cggcaggtcc gcctttgccc ccttagcggg aacgctgtcc gaaaggacgt ggtacaattg 3120
   ctcaaccggg ccgggtacag gtccaccggg tttccgcgcc gggagtggga ccttaacctt 3180
   caaagtcttt ttcttcgggc tctttccctg agcgggccgt tgagttttct ggagaactac 3240
   tccgtccccc gatgcatgcg catgacccgc ttgctcatcg cccggctttt tacccgagat 3300
   ggactgagtt tgtctgtctc gatggaccac cgacggcaaa cctggtgaat ttcctctcgt 3360
   cgtttgtcgg ggtatagacc gctggtcttc ccgttgatcg ttcccggcgg cgtctccaac 3420
   aggagacgcg ggggatacag gggagaaggc ctgcgggaac ggaggggtcg tacctctgcc 3480
   cgtttcccca tcgttcatcg gtggttttgg agacctagca agcttcgttc cgagagagac 3540
   tgtctcgagg gagcgatcgg ctcctgttgg ttctcgcgcg ccggcctccg agaatcgggt 3600
   gtggaagacc tcggccagcg ggattacagg cgagcccatt agatcctgac cgtcctcgca 3660
   tacgtagtcg tcttgtgtta gctcttcgcc aacatcttcc gttctgggtt ctggttgaag 3720
   tcccgatacg gagggaattg aaacgatctc atgttcccgt cccaccatga ccccgttctc 3780
   tccaaatagt agatcgtcag gctgactcga ggtgaccacc cgggccctgt gttcggcggc 3840
   cgcggcggcc gcgtccaaca ggtccattaa ctccaaagta tcaggcgacc ccgcgcgttg 3900
   gggtgtagag cgctgcatcg gcggcgtatc catcgcactg gggtgaattt agacgtaccc 3960
   gagttttcca aacgctctcg cagccttcaa aggattgcga ttgcggttgg tgagggagtt 4020
   ccaacagtac ttaaaacgtg ttgtgccccc ccctcgaccg catatttcct ccccgtgtcg 4080
   tcaccgtgta aatattctta atgataagac gatgtagtga ttggacgaga ctcgaggcgg 4140
   gaagttcatg gaccatagta tgcgtttaag gagagaccgc tggttggcga tgtacgcccg 4200
   gtgtctattt ccgcatacct tacaacatca taacaaggga taccagacat gtgaatttca 4260
   tttacatatg tttaaataac aaccaatcat cgtgtgtcta cagacgatat atataataca 4320
   taaacacaat tggggttgtc tcacatgcaa aacatcttat ataacacggg ttgtttccac 4380
   ccatccggca tctagttaat caaatgcacg tcgacggtgt gtttgggtcc ctctccgtcg 4440
   tcattacgtt cgctcaatca acaagcgtat acaccaccac ccctcccaac 4490
<210> 2
   <211> 1310
   <212> PRT
   <213> Varicell azoster
<400> 2
   Met Asp Thr Pro Pro Met Gln Arg Ser Thr Pro Gln Arg Ala Gly Ser
   1 5 10 15
   Pro Asp Thr Leu Glu Leu Met Asp Leu Leu Asp Ala Ala Ala Ala Ala
   20 25 30
   Ala Glu His Arg Ala Arg Val Val Thr Ser Ser Gln Pro Asp Asp Leu
   35 40 45
   Leu Phe Gly Glu Asn Gly Val Met Val Gly Arg Glu His Glu Ile Val
   50 55 60
   Ser Ile Pro Ser Val Ser Gly Leu Gln Pro Glu Pro Arg Thr Glu Asp
   65 70 75 80
   Val Gly Glu Glu Leu Thr Gln Asp Asp Tyr Val Cys Glu Asp Gly Gln
   85 90 95
   Asp Leu Met Gly Ser Pro Val Ile Pro Leu Ala Glu Val Phe His Thr
   100 105 110
   Arg Phe Ser Glu Ala Gly Ala Arg Glu Pro Thr Gly Ala Asp Arg Ser
   115 120 125
   Leu Glu Thr Val Ser Leu Gly Thr Lys Leu Ala Arg Ser Pro Lys Pro
   130 135 140
   Pro Met Asn Asp Gly Glu Thr Gly Arg Gly Thr Thr Pro Pro Phe Pro
   145 150 155 160
   Gln Ala Phe Ser Pro Val Ser Pro Ala Ser Pro Val Gly Asp Ala Ala
   165 170 175
   Gly Asn Asp Gln Arg Glu Asp Gln Arg Ser Ile Pro Arg Gln Thr Thr
   180 185 190
   Arg Gly Asn Ser Pro Gly Leu Pro Ser Val Val His Arg Asp Arg Gln
   195 200 205
   Thr Gln Ser Ile Ser Gly Lys Lys Pro Gly Asp Glu Gln Ala Gly His
   210 215 220
   Ala His Ala Ser Gly Asp Gly Val Val Leu Gln Lys Thr Gln Arg Pro
   225 230 235 240
   Ala Gln Gly Lys Ser Pro Lys Lys Lys Thr Leu Lys Val Lys Val Pro
   245 250 255
   Leu Pro Ala Arg Lys Pro Gly Gly Pro Val Pro Gly Pro Val Glu Gln
   260 265 270
   Leu Tyr His Val Leu Ser Asp Ser Val Pro Ala Lys Gly Ala Lys Ala
   275 280 285
   Asp Leu Pro Phe Glu Thr Asp Asp Thr Arg Pro Arg Lys His Asp Ala
   290 295 300
   Arg Gly Ile Thr Pro Arg Val Pro Gly Arg Ser Ser Gly Gly Lys Pro
   305 310 315 320
   Arg Ala Phe Leu Ala Leu Pro Gly Arg Ser His Ala Pro Asp Pro Ile
   325 330 335
   Glu Asp Asp Ser Pro Val Glu Lys Lys Pro Lys Ser Arg Glu Phe Val
   340 345 350
   Ser Ser Ser Ser Ser Ser Ser Ser Trp Gly Ser Ser Ser Glu Asp Glu
   355 360 365
   Asp Asp Glu Pro Arg Arg Val Ser Val Gly Ser Glu Thr Thr Gly Ser
   370 375 380
   Arg Ser Gly Arg Glu His Ala Pro Ser Pro Ser Asn Ser Asp Asp Ser
   385 390 395 400
   Asp Ser Asn Asp Gly Gly Ser Thr Lys Gln Asn Ile Gln Pro Gly Tyr
   405 410 415
   Arg Ser Ile Ser Gly Pro Asp Pro Arg Ile Arg Lys Thr Lys Arg Leu
   420 425 430
   Ala Gly Glu Pro Gly Arg Gln Arg Gln Lys Ser Phe Ser Leu Pro Arg
   435 440 445
   Ser Arg Thr Pro Ile Ile Pro Pro Val Ser Gly Pro Leu Met Met Pro
   450 455 460
   Asp Gly Ser Pro Trp Pro Gly Ser Ala Pro Leu Pro Ser Asn Arg Val
   465 470 475 480
   Arg Phe Gly Pro Ser Gly Glu Thr Arg Glu Gly His Trp Glu Asp Glu
   485 490 495
   Ala Val Arg Ala Ala Arg Ala Arg Tyr Glu Ala Ser Thr Glu Pro Val
   500 505 510
   Pro Leu Tyr Val Pro Glu Leu Gly Asp Pro Ala Arg Gln Tyr Arg Ala
   515 520 525
   Leu Ile Asn Leu Ile Tyr Cys Pro Asp Arg Asp Pro Ile Ala Trp Leu
   530 535 540
   Gln Asn Pro Lys Leu Thr Gly Val Asn Ser Ala Leu Asn Gln Phe Tyr
   545 550 555 560
   Gln Lys Leu Leu Pro Pro Gly Arg Ala Gly Thr Ala Val Thr Gly Ser
   565 570 575
   Val Ala Ser Pro Val Pro His Val Gly Glu Ala Met Ala Thr Gly Glu
   580 585 590
   Ala Leu Trp Ala Leu Pro His Ala Ala Ala Ala Val Ala Met Ser Arg
   595 600 605
   Arg Tyr Asp Arg Ala Gln Lys His Phe Ile Leu Gln Ser Leu Arg Arg
   610 615 620
   Ala Phe Ala Gly Met Ala Tyr Pro Glu Ala Thr Gly Ser Ser Pro Ala
   625 630 635 640
   Ala Arg Ile Ser Arg Gly His Pro Ser Pro Thr Thr Pro Ala Thr Gln
   645 650 655
   Thr Pro Asp Pro Gln Pro Ser Ala Ala Ala Arg Ser Leu Ser Val Cys
   660 665 670
   Pro Pro Asp Asp Arg Leu Arg Thr Pro Arg Lys Arg Lys Ser Gln Pro
   675 680 685
   Val Glu Ser Arg Ser Leu Leu Asp Lys Ile Arg Glu Thr Pro Val Ala
   690 695 700
   Asp Ala Arg Val Ala Asp Asp His Val Val Ser Lys Ala Lys Arg Arg
   705 710 715 720
   Val Ser Glu Pro Val Thr Ile Thr Ser Gly Pro Val Val Asp Pro Pro
   725 730 735
   Ala Val Ile Thr Met Pro Leu Asp Gly Pro Ala Pro Asn Gly Gly Phe
   740 745 750
   Arg Arg Ile Pro Arg Gly Ala Leu His Thr Pro Val Pro Ser Asp Gln
   755 760 765
   Ala Arg Lys Ala Tyr Cys Thr Pro Glu Thr Ile Ala Arg Leu Val Asp
   770 775 780
   Asp Pro Leu Phe Pro Thr Ala Trp Arg Pro Ala Leu Ser Phe Asp Pro
   785 790 795 800
   Gly Ala Leu Ala Glu Ile Ala Ala Arg Arg Pro Gly Gly Gly Asp Arg
   805 810 815
   Arg Phe Gly Pro Pro Ser Gly Val Glu Ala Leu Arg Arg Arg Cys Ala
   820 825 830
   Trp Met Arg Gln Ile Pro Asp Pro Glu Asp Val Arg Leu Leu Ile Ile
   835 840 845
   Tyr Asp Pro Leu Pro Gly Glu Asp Ile Asn Gly Pro Leu Glu Ser Thr
   850 855 860
   Leu Ala Thr Asp Pro Gly Pro Ser Trp Ser Pro Ser Arg Gly Gly Leu
   865 870 875 880
   Ser Val Val Leu Ala Ala Leu Ser Asn Arg Leu Cys Leu Pro Ser Thr
   885 890 895
   His Ala Trp Ala Gly Asn Trp Thr Gly Pro Pro Asp Val Ser Ala Leu
   900 905 910
   Asn Ala Arg Gly Val Leu Leu Leu Ser Thr Arg Asp Leu Ala Phe Ala
   915 920 925
   Gly Ala Val Glu Tyr Leu Gly Ser Arg Leu Ala Ser Ala Arg Arg Arg
   930 935 940
   Leu Leu Val Leu Asp Ala Val Ala Leu Glu Arg Trp Pro Gly Asp Gly
   945 950 955 960
   Pro Ala Leu Ser Gln Tyr His Val Tyr Val Arg Ala Pro Ala Arg Pro
   965 970 975
   Asp Ala Gln Ala Val Val Arg Trp Pro Asp Ser Ala Val Thr Glu Gly
   980 985 990
   Leu Ala Arg Ala Val Phe Ala Ser Ser Arg Thr Phe Gly Pro Ala Ser
   995 1000 1005
   Phe Ala Arg Ile Glu Thr Ala Phe Ala Asn Leu Tyr Pro Gly Glu
   1010 1015 1020
   Gln Pro Leu Cys Leu Cys Arg Gly Gly Asn Val Ala Tyr Thr Val
   1025 1030 1035
   Cys Thr Arg Ala Gly Pro Lys Thr Arg Val Pro Leu Ser Pro Arg
   1040 1045 1050
   Glu Tyr Arg Gln Tyr Val Leu Pro Gly Phe Asp Gly Cys Lys Asp
   1055 1060 1065
   Leu Ala Arg Gln Ser Arg Gly Leu Gly Leu Gly Ala Ala Asp Phe
   1070 1075 1080
   Val Asp Glu Ala Ala His Ser His Arg Ala Ala Asn Arg Trp Gly
   1085 1090 1095
   Leu Gly Ala Ala Leu Arg Pro Val Phe Leu Pro Glu Gly Arg Arg
   1100 1105 1110
   Pro Gly Ala Ala Gly Pro Glu Ala Gly Asp Val Pro Thr Trp Ala
   1115 1120 1125
   Arg Val Phe Cys Arg His Ala Leu Leu Glu Pro Asp Pro Ala Ala
   1130 1135 1140
   Glu Pro Leu Val Leu Pro Pro Val Ala Gly Arg Ser Val Ala Leu
   1145 1150 1155
   Tyr Ala Ser Ala Asp Glu Ala Arg Asn Ala Leu Pro Pro Ile Pro
   1160 1165 1170
   Arg Val Met Trp Pro Pro Gly Phe Gly Ala Ala Glu Thr Val Leu
   1175 1180 1185
   Glu Gly Ser Asp Gly Thr Arg Phe Val Phe Gly His His Gly Gly
   1190 1195 1200
   Ser Glu Arg Pro Ala Glu Thr Gln Ala Gly Arg Gln Arg Arg Thr
   1205 1210 1215
   Ala Asp Asp Arg Glu His Ala Leu Glu Pro Asp Asp Trp Glu Val
   1220 1225 1230
   Gly Cys Glu Asp Ala Trp Asp Ser Glu Glu Gly Gly Gly Asp Asp
   1235 1240 1245
   Gly Asp Ala Pro Gly Ser Ser Phe Gly Val Ser Ile Val Ser Val
   1250 1255 1260
   Ala Pro Gly Val Leu Arg Asp Arg Arg Val Gly Leu Arg Pro Ala
   1265 1270 1275
   Val Lys Val Glu Leu Leu Ser Ser Ser Ser Ser Ser Glu Asp Glu
   1280 1285 1290
   Asp Asp Val Trp Gly Gly Arg Gly Gly Arg Ser Pro Pro Gln Ser
   1295 1300 1305
   Arg Gly
   1310
<210> 3
   <211> 16
   <212> PRT
   <213> Artificial
<220>
   <223> epi t ope
<400> 3
   Pro Gly Tyr Arg Ser Ile Ser Gly Pro Asp Pro Arg Ile Arg Lys Thr
   1 5 10 15
<210> 4
   <211> 15
   <212> PRT
   <213> Artificial
<220>
   <223> epitope
<400> 4
   Lys Arg Leu Ala Gly Glu Pro Gly Arg Gln Arg Gln Lys Ser Phe
   1 5 10 15
<210> 5
   <211> 15
   <212> PRT
   <213> Artificial
<220>
   <223> epi t ope
<400> 5
   Ser Leu Pro Arg Ser Arg Thr Pro Ile Ile Pro Pro Val Ser Gly
   1 5 10 15
<210> 6
   <211> 15
   <212> DNA
   <213> Artificial
<220>
   <223> primer
<400> 6
   aagctttttt ttttv v 15
<210> 7
   <211> 22
   <212> DNA
   <213> Artificial
<220>
   <223> primer
<400> 7
   act caaaggg aaacgt gt ct ct 22
<210> 8
   <211> 21
   <212> DNA
   <213> Artificial
<220>
   <223> primer
<400> 8
   cggt gt aggc t ggaat agac t 21
<210> 9
   <211> 21
   <212> DNA
   <213> Artificial
<220>
   <223> primer
<400> 9
   ctccgccatg caatttccac t 21
<210> 10
   <211> 22
   <212> DNA
   <213> Artificial
<220>
   <223> primer
<400> 10
   gt gacaacaa t gt gact cca ct 22
<210> 11
   <211> 20
   <212> DNA
   <213> Artificial
<220>
   <223> primer
<400> 11
   gccttcatgc aaccgaagt a 20
<210> 12
   <211> 24
   <212> DNA
   <213> Artificial
<220>
   <223> primer
<400> 12
   tccatgacaa ctttggcatt gtgg 24
<210> 13
   <211> 24
   <212> DNA
   <213> Artificial
<220>
   <223> primer
<400> 13
   gttgctgttg aagtcgcagg agac 24
<210> 14
   <211> 20
   <212> DNA
   <213> Artificial
<220>
   <223> primer
<400> 14
   cgctggaaga agtccatcaa 20
<210> 15
   <211> 20
   <212> DNA
   <213> Artificial
<220>
   <223> primer
<400> 15
   gggct aacag tgtagtcgta a 20
<210> 16
   <211> 20
   <212> DNA
   <213> Artificial
<220>
   <223> primer
<400> 16
   tttgagacct tcaacacccc 20
<210> 17
   <211> 20
   <212> DNA
   <213> Artificial
<220>
   <223> primer
<400> 17
   acgatttccc tctcagctgt 20
<210> 18
   <211> 74
   <212> PRT
   <213> Artificial
<220>
   <223> epitope
<400> 18
   Gly Pro Val Glu Gln Leu Tyr His Val Leu Ser Asp Ser Val Pro Ala
   1 5 10 15
   Lys Gly Ala Lys Ala Asp Leu Pro Phe Glu Thr Asp Asp Thr Arg Pro
   20 25 30
   Arg Lys His Asp Ala Arg Gly Ile Thr Pro Arg Val Pro Gly Arg Ser
   35 40 45
   Ser Gly Gly Lys Pro Arg Ala Phe Leu Ala Leu Pro Gly Arg Ser His
   50 55 60
   Ala Pro Asp Pro Ile Glu Asp Asp Ser Pro
   65 70
<210> 19
   <211> 15
   <212> PRT
   <213> Artificial
<220>
   <223> synthetic peptide
<400> 19
   Gly Arg Ser Ser Gly Gly Lys Pro Arg Ala Phe Leu Ala Leu Pro
   1 5 10 15
<210> 20
   <211> 15
   <212> PRT
   <213> Artificial
<220>
   <223> synthetic peptide
<400> 20
   Asp Thr Arg Pro Arg Lys His Asp Ala Arg Gly Ile Thr Pro Arg
   1 5 10 15